(19) Europäisches Patentamt
European Patent Office
Office européen des brevets

(11) **EP 3 626 727 B1**

(12) **EUROPEAN PATENT SPECIFICATION**

(45) Date of publication and mention of the grant of the patent:
**08.10.2025 Bulletin 2025/41**

(21) Application number: **18802073.9**

(22) Date of filing: **14.05.2018**

(51) International Patent Classification (IPC):
***C07K 5/093*** (2006.01) ***C07K 5/113*** (2006.01)
***C07K 7/06*** (2006.01) ***A61K 38/06*** (2006.01)
***A61K 38/07*** (2006.01) ***A61K 38/08*** (2019.01)
***G01N 33/68*** (2006.01) ***A61P 1/00*** (2006.01)
***A61P 9/12*** (2006.01) ***C07K 5/065*** (2006.01)
***C07K 5/068*** (2006.01) ***C07K 5/072*** (2006.01)
***C07K 5/087*** (2006.01) ***A61P 3/00*** (2006.01)
***A61P 25/00*** (2006.01)

(52) Cooperative Patent Classification (CPC):
**C07K 5/06121; A61P 1/00; A61P 3/04; A61P 3/10; A61P 3/14; A61P 5/00; A61P 5/14; A61P 9/12; A61P 19/10; A61P 25/04; A61P 25/06; A61P 25/08; A61P 25/28; C07K 5/06078; C07K 5/06086;**
(Cont.)

(86) International application number:
**PCT/BR2018/050156**

(87) International publication number:
**WO 2018/209415 (22.11.2018 Gazette 2018/47)**

(54) **COMPOUND, SYNTHETIC INTERMEDIATE, USE, PHARMACEUTICAL COMPOSITION, AND NEUROMODULATORY THERAPEUTIC METHOD**

VERBINDUNG, SYNTHETISCHES ZWISCHENPRODUKT, VERWENDUNG, PHARMAZEUTISCHE ZUSAMMENSETZUNG UND NEUROMODULATORISCHES THERAPIEVERFAHREN

COMPOSÉ, PRODUIT INTERMÉDIAIRE DE SYNTHÈSE, UTILISATION, COMPOSITION PHARMACEUTIQUE ET MÉTHODE THÉRAPEUTIQUE DE NEUROMODULATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.05.2017 BR 102017010169**
**11.10.2017 PCT/BR2017/050314**

(43) Date of publication of application:
**25.03.2020 Bulletin 2020/13**

(73) Proprietors:
- **Remer Consultores Assessoria Empresarial Ltda.**
  **20010-020 Rio De Janeiro (BR)**
- **Proteimax Biotecnologia Ltda**
  **05581001 São Paulo (BR)**

(72) Inventors:
- **REMER, Ricardo Amaral**
  **22471-001 Rio De Janeiro (BR)**
- **HEIMANN, Andrea Sterman**
  **04531-000 Sao Paulo (BR)**

(74) Representative: **Hübscher & Partner Patentanwälte GmbH**
**Spittelwiese 4**
**4020 Linz (AT)**

(56) References cited:
**WO-A1-2012/131676**
**WO-A1-2014/170347**
**WO-A1-2018/068120**
**WO-A2-2011/146514**
**WO-A2-2013/040142**
**CN-A- 104 211 769**
**US-A- 5 888 794**
**US-A1- 2004 258 695**
**US-A1- 2008 146 510**
**US-A1- 2013 330 335**
**US-A1- 2015 297 669**
**US-B2- 8 092 994**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- ESZTER SZLAVICZ ET AL: "Further Characterization of Hemopressin Peptide Fragments in the Opioid and Cannabinoid Systems :", ANESTHESIA AND ANALGESIA., vol. 121, no. 6, 1 December 2015 (2015-12-01), US, pages 1488 - 1494, XP055476031, ISSN: 0003-2999, DOI: 10.1213/ANE.0000000000000964
- JAMES P. TAM ET AL: "Stereospecific Pseudoproline Ligation of N-Terminal Serine, Threonine, or Cysteine-Containing Unprotected Peptides", JOURNAL OF THE AMERICAN CHEMICAL SOCIETY, vol. 121, no. 39, 1 October 1999 (1999-10-01), US, pages 9013 - 9022, XP055764205, ISSN: 0002-7863, DOI: 10.1021/ja991153t
- SCHARN D ET AL: "Sequential nucleophilic substitution on halogenated triazines, pyrimidines, and purines: A novel approach to cyclic peptidomimetics", JOURNAL OF ORGANIC CHEMISTRY, AMERICAN CHEMICAL SOCIETY, WASHINGTON, vol. 66, 1 January 2001 (2001-01-01), pages 507 - 513, XP002241440, ISSN: 0022-3263, DOI: 10.1021/JO005631Q
- JOVENCIO HILARIO ET AL: "Optical Spectroscopic Investigations of Model [beta]-Sheet Hairpins in Aqueous Solution", JOURNAL OF THE AMERICAN CHEMICAL SOCIETY, vol. 125, no. 25, 1 June 2003 (2003-06-01), US, pages 7562 - 7574, XP055764697, ISSN: 0002-7863, DOI: 10.1021/ja030039e
- MATTHEW BOGYO: "Metalloproteases see the light", NATURE CHEMICAL BIOLOGY, vol. 2, no. 5, 1 May 2006 (2006-05-01), New York, pages 229 - 230, XP055764699, ISSN: 1552-4450, DOI: 10.1038/nchembio0506-229
- WAKIMASU M ET AL: "4-METHOXY-2,3,6-TRIMETHYLBENZENESULFONYL (MTR): A NEW AMINO AND IMIDAZOLE PROTECTING GROUP IN PEPTIDE SYNTHESIS", CHEMICAL AND PHARMACEUTICAL BULLETIN, PHARMACEUTICAL SOCIETY OF JAPAN, JP, vol. 30, no. 8, 1 January 1982 (1982-01-01), pages 2766 - 2779, XP001154511, ISSN: 0009-2363
- YOON S S ET AL: "Sequence-Selective Peptide Binding with a Synthetic Receptor", TETRAHEDRON, ELSEVIER SIENCE PUBLISHERS, AMSTERDAM, NL, vol. 51, no. 2, 9 January 1995 (1995-01-09), pages 567 - 578, XP004104933, ISSN: 0040-4020, DOI: 10.1016/0040-4020(94)00916-I
- ABIKO T ET AL: "Identification and synthesis of a heptapeptide in uremic fluid", BIOCHEMICAL AND BIOPHYSICAL RESEARCH COMMUNICATIONS, ELSEVIER, AMSTERDAM NL, vol. 84, no. 1, 14 September 1978 (1978-09-14), pages 184 - 194, XP024773073, ISSN: 0006-291X, [retrieved on 19780914], DOI: 10.1016/0006-291X(78)90280-2
- MATTEO CONZA ET AL: "Selective Binding of Two-Armed Diketopiperazine Receptors to Side-Chain-Protected Peptides", J ORG CHEM, vol. 67, 21 March 2002 (2002-03-21), pages 2696 - 2698, XP055764743
- KATHERINE T. BARGLOW ET AL: "Substrate Mimicry in an Activity-Based Probe That Targets the Nitrilase Family of Enzymes", ANGEWANDTE CHEMIE INTERNATIONAL EDITION, vol. 45, no. 44, 13 November 2006 (2006-11-13), pages 7408 - 7411, XP055764747, ISSN: 1433-7851, DOI: 10.1002/anie.200603187
- KATHERINE T. BARGLOW ET AL: "Substrate Mimicry in an Activity-Based Probe That Targets the Nitrilase Family of Enzymes - Supporting Information", ANGEWANDTE CHEMIE INTERNATIONAL EDITION, vol. 45, no. 44, 13 November 2006 (2006-11-13), pages 7408 - 7411, XP055764751, DOI: 10.1002/anie.200603187
- MICHAL PECHAR ET AL: "Poly(ethylene glycol)-Based Polymer Carrier of Doxorubicin Degradable by Both Enzymatic and Chemical Hydrolyses", COLLECTION OF CZECHOSLOVAK CHEMICAL COMMUNICATIONS, vol. 70, no. 3, 1 January 2005 (2005-01-01), CZ, pages 327 - 338, XP055764745, ISSN: 0010-0765, ISBN: 978-80-86241-25-8, DOI: 10.1135/cccc20050327
- GOMES, I. ET AL: "Novel endogenous peptide agonists of cannabinoid receptors", FASEB JOURNAL, vol. 23, no. 9, 1 September 2009 (2009-09-01), pages 3020 - 3029, XP055186735, ISSN: 0892-6638, DOI: 10.1096/fj.09-132142
- HEIMANN, A.S. ET AL: "Hemopressin is an inverse agonist of CB1 cannabinoid receptors", PROC.NATL.ACAD.SCI, vol. 104, no. 51, 18 December 2007 (2007-12-18), pages 20588 - 20593, XP002614960, ISSN: 0027-8424, DOI: 10.1073/pnas.0706980105
- GOMES, I. ET AL.: "Hemoglobin-derived peptides as novel types of biactive signaling molecules", THE AAPS JOURNAL, vol. 12, no. 4, 2 September 2010 (2010-09-02), pages 658 - 669, XP035719135, DOI: 10.1208/s12248-010-9217-x


(52) Cooperative Patent Classification (CPC): (Cont.)
**C07K 5/0812; C07K 5/0819; C07K 5/1021; C07K 7/06; G01N 33/948;** A61K 38/00

## Description

### Field of the Invention

The present invention is in the fields of pharmaceutical sciences, medicine, chemistry and biotechnology. The invention is set out in the appended set of claims.

### Background of the Invention

The peptidic compound of the invention is surprisingly stable at extreme temperatures, does not pose the problem of fibril formation and provides for ease of handling in the preparation of products of pharmaceutical interest, including ligands for diagnostics and pharmaceutical compositions.

The peptidic compound which is closest to the present invention is hemopressin and its larger size variants with biological activity. However, hemopressin presents technical difficulties such as instability and tendency to form fibrils, which limit its use in pharmaceutical preparations (Bomar MG & Galande AK, Modulation of the cannabinoid receptors by hemopressin peptides. 520-524). These limitations have diminished initial enthusiasm with hemopressin: its natural tendency for aggregation/fibril formation substantially limits the range of possible concentrations at both the synthesis, pharmaceutical preparation and therapeutic use. The same literature (Bomar & Galande) goes so far as to speculate that hemopressin self-assembly may be physiologically relevant and potentially pathogenic or neurotoxic, analogously to the amyloid peptidic fibrils associated with Alzheimer's disease, Parkinson's disease and type II diabetes.

The results of the present invention are surprising in view of the closest background, and the present inventors are not aware of any report or suggestion in the literature that the peptidic compound of the invention would be more stable and more easily manipulable than Hemopressin, thus being in relation to this, an unexpected improvement and of unexpected magnitude.

The cannabinoid system, which comprises the CB1 and CB2 receptors and their endogenous ligands, acts on various metabolic functions, including control of food intake, energy and/or lipid metabolism, regulation of intestinal motility, immune system, in the balance of the calcium cycle, among others. Cannabinoid receptors are widely expressed in the brain, including cortex, hippocampus, amygdala, pituitary, and hypothalamus. CB receptors, particularly CB1, have already been identified in numerous peripheral organs and tissues, including the thyroid gland, adrenal gland, reproductive organs, adipose tissue, liver, muscle, and gastrointestinal tract.

Bomar & Galande's article also shows that N-extended versions of hemopressin, such as RVD-Hp, VD-Hp, have shown an agonist effect on the CB1 receptor, and that, therefore, a difference of only two or three amino acids appears to interfere if the peptide exhibits antagonistic or agonist effect, so as to produce opposite effects on CB1. In addition, the signaling pathways for the different known peptides are different from the classical pathway mediated by the G protein, so that no extrapolation is reliable in light of the literature reports available so far. Similar conflicting effects may also occur with respect to the CB2 receptor.

The literature also clearly shows that while hemopressin and said known extended-size variants have therapeutic activity, peptidic compounds of size less than 6 amino acids do not provide therapeutic effect (Szlavicz et al, 2015; Dvorácskó et al, 2016; Bomar & Galande, 2013; Bauer et al, 2012).

The present invention surprisingly proves the opposite: in addition to providing more intense therapeutic effects in the known uses for hemopressin and extended-size variants, it also provides other uses not previously known or suggested. In addition, small changes in peptide sizes may not only lead to substantial and unexpected changes in therapeutic effects, but may also lead to the complete absence of effects.

It should also be noted that studies with peptides resembling hemopressin (pepcans) have been facing several challenges. For example, manipulation of said larger peptides has been hindered by fibril aggregation/formation. In addition, the metabolic stability of said peptides *in vivo* is limited and data from the respective pharmacological experiments are difficult to interpret without pharmacokinetic analysis, leading to confusion in the literature. Although there are studies with hemopressin (Hp) and RV-Hp administration, the only endogenous peptides naturally present at physiological levels appear to be RVD-Hp (pepcan 12) and pepcan 23.

In this context, various compounds have already been detected in the art as having cannabinoid receptor modulation activity. Among these, some target the development of drugs for weight reduction and thinning of the waist, such as rimonabant. However, this compound was subsequently associated with increased occurrence of psychiatric disorders in humans and was removed from the world market.

The compound of the invention additionally provides the advantage of being a good candidate as substitute to the cannabinoid compounds, such as cannabidiol. Cannabidiol, despite its proven effects, has been facing regulatory problems due to its origin, the *Cannabis sativa* plant. The present invention provides an additional therapeutic approach for patients suffering from seizures and having difficulty obtaining drugs, being based on a peptidic compound and not using derivatives of *Cannabis sativa.* The results showed that the compound of the invention, when used as an

anticonvulsant, provides other surprising technical advantages in use, including increased therapeutic effect, oral use, lower dosage, less occurrence of side effects such as prostration and nasal bleeding, among other technical advantages.

Pilocarpine (commonly called "Pilo") is an alkaloid extracted from the leaves of the jaborandi plant (*Pilocarpus jaborandi*), a plant used for centuries by the Tupi-Guarani Indians who inhabit Brazil and take advantage of their properties to produce sweat and saliva. Pilocarpine is a nonspecific muscarinic agonist, slowly degraded and without effects on nicotinic receptors and was introduced in clinical practice by the Brazilian physician Sifrônio Coutinho, in 1874, through extracts from the jaborandi leaf to obtain a diaphoretic (sweat production) and silagogue (production of saliva) effect.

Despite its pharmaceutical properties, pilocarpine at high concentrations induces the occurrence of convulsions, being used as an experimental model therefor. Pilocarpine-induced seizures lead to neurotoxicity at the cellular level and may be related to increased cerebral oxidative stress and changes in the concentration of certain amino acids (Santos et al, 2011).

Administration of pilocarpine in this experimental model leads to severe brain injury, neurotoxicity and usually culminates in the death of animals. However, before this outcome, pilocarpine causes cholinergic alterations capable of inducing *status epilepticus* (SE) associated with convulsive stereotyped movements. Pilocarpine is able to induce status epilepticus either administered directly into the brain or intraperitoneally. This substance was used in some experiments described in this patent application, its harmful neuronal/encephalic effects having been inhibited or prevented by the composition of the present invention: substantial part of the animals subjected to these experiments had no symptoms related to brain lesions and survived without apparent damage, in contrast to groups of animals treated with other known substances.

Much of the understanding of the mechanisms of epilepsy comes from studies in animal experimental models, especially in rats and mice. In this context, the administration of pilocarpine in rodents mimics epilepsy (ELT) in humans and is generally referred to as the "pilocarpine model". This model was developed in 1983 by Turski et al. and is now one of the most widely used models of epilepsy, since its histological, biochemical, pharmacological, electrophysiological and behavioral characteristics (Turski et al., 1983) similarly reproduce those found in human carriers of ELT.

The pilocarpine model is also useful for evidencing changes in muscarinic receptors, such as the occurrence of salivation. Acetylcholine, through its muscarinic receptor (mAChRs) plays an important role in cognitive functions, such as learning and memory. mAChRs are receptors that form complexes with G protein-receptor in the cell membranes of certain neurons and other cells. They play several roles, including acting as the ultimate end receptor stimulated by acetylcholine released from postganglionic fibers in the parasympathetic nervous system.

Muscarinic receptors are so-called because they are more sensitive to muscarin than to nicotine. Its counterparts are nicotinic acetylcholine receptors (nAChRs), ion channels receptors that are also important in the autonomous nervous system. Many drugs and other substances (eg, pilocarpine and esco-polamine) manipulate these two distinct receptors acting as selective agonists or antagonists.

The mAChRs are among the most well characterized among the transmembrane receptors (7TM), being widely expressed in the central nervous system (CNS). Five mAChR subtypes were cloned (M1, M2, M3, M4 and M5) and are generally divided into two distinct classes based on signal transduction. mAChR M1, M3 and M5 are subtypes that signal through Gq/11 proteins and activate phospholipase-C and mobilize intracellular calcium. mAChR M2 and M4, however, predominate through Gi/o proteins inhibiting adenylate cyclase and reducing the intracellular concentration of cAMP. The predominant mAChR in the CNS is the M1 subtype, which is located in the cortex, hippocampus, striatum and thalamus, where it is found post-synaptic. M2 mAChRs are located predominantly in the brainstem and thalamus, although also in the cortex, hippocampus and striatum, where they control the release of acetylcholine. mAChRs of M3 and M5 are expressed at much lower levels than M1 or M2 mAChRs in the CNS, but M3 mAChRs are found in the cortex and hippocampus, while M5 mAChRs have a very discrete location in the *substantia nigra*. M4 mAChRs are found in many regions of the brain, including the cortex and hippocampus, but are more prominent in the striatum, where they are thought to play a role in the control of dopamine release and modulate locomotor activity.

Given the broad and varied expression profile of mAChRs in the CNS, it is not surprising that all subtypes have been evaluated as potential drug targets. Some of these targets are well accepted in the literature, such as the involvement of M1 in Alzheimer's disease, while others are relatively new, such as M5 that has been correlated with dependency and addiction to abuse drugs.

Multiple sclerosis is a neuroinflammatory disease of the central nervous system with a strong neurodegenerative component, and has a great impact on the health of the patients. The disease still has few satisfactory available therapeutic options, and the development of alternatives is highly desirable, as is the case of the present invention.

Multiple sclerosis is characterized by the destruction of oligodendro-cytes and neurons, resulting in heterogeneous and cumulative clinical symptoms. The currently available therapies are only partially effective and are primarily directed to the inflammatory phase of the disease. However, the neurodegenerative component of the disease is still the greatest challenge for new therapeutic approaches. The present invention is precisely intended to fill this important gap.

Although the exact etiology of the disease is still unclear, it is speculated that self-reactive T lymphocytes play a central role in its pathophysiology. The most common animal model of Multiple Sclerosis is experimental autoimmune

encephalomyelitis (EAE), because it shares many physiological and clinical aspects. There are several models of EAE that reflect different clinical, immunological and histological aspects of human multiple sclerosis. The active induction model of EAE in mice is robust and provides replicable results, being particularly useful in the search for therapeutic agents through the use of transgenic mice challenged by autoimmune neuroinflammation. The EAE model is often used as a 'proof of principle' of the efficacy of novel therapeutic strategies for Multiple Sclerosis.

Background searches have disclosed documents only partially relevant for the present invention. Such documents will be described below, being set forth herein solely for the purpose of serving as a basis for the state of the art, since none of them anticipates or even suggests any of the objects of the present invention.

Some studies presented by one of the present inventors in Novel Peptide Substrates for Endopeptidase 24.15 Neurolysin and Angiotensin Converting Enzyme (Vanessa Rioli, Fabio C. Gozzo, Andrea S. Heimann, Alessandra Linardi, Jose E. Krieger, Claudio S. Shida, Paulo C. Almeida, Stephen Hyslop, Marcos N. Eberlin, Emer S. Ferro; March 7, 2003) demonstrate an effective technique of "screening" new peptides. The present invention differs from that document, among other reasons, because it provides a different molecular entity. It also provides a more stable and more therapeutically effective compound. In addition, it provides a novel therapeutic approach to neuromodulation, neuroprotection, analgesia, convulsions or the treatment of multiple sclerosis, the results of which have been surprising.

Gomes et al (Gomes I, Grushko JS, Golebiewska U, Hoogendoorn S, Gupta A, Heimann AS, Ferro ES, Scarlata S, Fricker LD, and Devi LA Novel endogenous peptide agonists of cannabinoid receptors FASEB J. 2009 Sep; 23 (9): 3020-3029) is another scientific publication which has as one of the authors the co-inventor in the present invention (Heimann). Said co-inventor, when tests were proposed by the other co-inventor in order to verify the hypothesis of the the present invention, was surprised by the hypothesis - which itself reveals the non-obviousness thereof. It should be noted that Gomes et al was published in 2009, while the priority of the present invention is 2017, ie, even more than seven years after the publication of Gomes et al and practically ten years after the discovery of hemopressin (Hp) by the inventor herself (Heimann et al., Proc Natl Acad Sci USA, 2007 Dec 18; 104 (51): 20588-93), not only she but no other researcher in the world had published or suggested the objects of the present invention.

Furthermore, Gomes et al. disclose the natural presence of certain endocannabinoids in the brain of mice (ie, local expression) having found N-extended versions of Hp. In addition, one of the conclusions of Gomes et al is that the N-extended versions found in the brain (RVD-Hp$\alpha$ and VD-Hp$\alpha$) have *opposite* function to that of the peptide of the present invention, this being antagonist/inverse agonist and those being agonist of cannabinoid receptors. Therefore, Gomes et al neither disclose or suggest the use of any endocannabinoid in the control of seizures or on neuromodulation, nor pharmaceutical compositions comprising any of these entities - much less the use of the peptide of the invention, any of the claimed uses, or oral compositions comprising the same. Consequently, not only Gomes et al do not disclose the use of the peptide of the invention, but the person skilled in the art, from reading Gomes et al., would not be motivated to: (i) synthesize the peptidic compound of the invention; (ii) expect its stability to be substantially greater than that of Hemopressin; (iii) expect the therapeutic action to be greater than that of Hemopressin; (iv) test it for the other uses indicated herein with reasonable expectation of success; or (v) prepare oral pharmaceutical compositions for neuromodulation, neuroprotection, seizure or multiple sclerosis treatment. In this context, the co-inventor herself did not see much sense in such approach before performing the tests suggested by the other co-inventor. The compound of the invention is novel and has surprising properties; in previously known uses, is a substantial and surprising improvement; in previously unknown uses, it is novel and surprising, as it is also surprising that an oral pharmaceutical composition containing a peptide has a neuromodulatory action. One skilled in the art would not expect said peptidic compound of the invention to have greater stability and resist the gastrointestinal tract and interact in order to produce obtained the neuromodulation/neuroprotection effects.

Document WO 2014/008567 has as one of the inventors the co-inventor in the present invention (Heimann). It discloses peptidic compounds and compositions useful for treating metabolic disorders comprising obesity, diabetes, systemic arterial hypertension (or disease, condition related and/or associated comorbidities); prevention of overweight; regulation of appetite; induction of satiety; prevention of weight gain after successful weight loss; increased energy consumption; aesthetic weight reduction; or bulimia. The compounds of said document interact with CB receptors, but are of different chemical structure than those of the present invention. Furthermore, no report or suggestion of use of the compound of the invention as a neuromodulator, neuroprotectant, anticonvulsant or as an entity useful in the treatment of multiple sclerosis is made in said document, because this approach was neither imagined by the inventors nor was it obvious from the state of the art.

Document WO 2011/011847 (=US2015/297669, US9,452,196, EP2459207) has as one of the inventors the inventor of the present invention (Heimann). This document only discloses the use of hemopressin (Hp) for the control of diabetes and obesity/weight gain and is limited to Hp and larger derivatives such as DV-Hp, PVD-Hp, RVD-Hp. The present invention differs from said document, among other reasons, for disclosing a different compound, which provides benefits when compared to hemopressin, including more ease of preparation, greater stability and biological activity, among other advantages. In addition, the compound of the invention provides other therapeutic uses not disclosed or suggested in WO 2011/011847: the test performed by the present inventors showed surprising results, especially in regard

to neuromodulation, neuroprotection, inhibition of seizures, and of multiple sclerosis symptoms, facts not described or suggested in said document. In addition, said co-inventor of WO 2011/011847, when the other co-inventor proposed tests to verify the hypothesis of the present invention, was struck by the hypothesis - which in itself reveals the non-obviousness thereof. Document WO 2011/011847 was published in 2011, while the priority of the present invention is 2017, ie even more than five years after the publication of the earliest precedent and practically ten years after the discovery of hemopressin by the co-inventor herself (Heimann et al., Proc Natl Acad Sci USA, 2007 Dec 18; 104 (51): 20588-93), not only she but no other researcher in the world had published or suggested the objects of the present invention. Finally, document WO 2011/011847 focuses on the action on receptors present in adipose tissue, which are outside the brain and not in brain receptors.

It is also important to note that the literature makes no mention to hemopressin, much less the peptide of the present invention, for the control of seizures. The use of hemopressin in seizure control has not been disclosed or suggested in the prior art and is the subject matter of another co-pending application of the same inventors, PCT/BR2017/050313. What the literature reveals are other molecular entities and which are CB1 receptor agonists, while the peptide of the invention is an inverse agonist and antagonist (modulator), which makes the results of the present invention even more surprising.

Document WO 2013/021196 discloses the use of hemopressin as an agent that interferes with the differentiation of oligodentrites. The present invention differs from said document, among other reasons, in that it reveals other compounds, other uses and provides benefits when compared to hemopressin, including more ease of preparation, greater stability and biological activity, among other advantages. In addition, the tests performed by the inventors showed surprising results, particularly in regard to neuromodulation, neuroprotection, inhibition of the occurrence of seizures and/or symptoms of multiple sclerosis with the compounds of the invention, which are neither described nor suggested in said document.

Other references of scientific literature which circumscribe the invention, but without anticipating or suggesting it, include the following documents.

ASPRONI, B. et al. Novel pyrrolocycloalkylpyrazole analogues as CB1 ligands. Chemical Biology and Drug Design, p. 1-13, 2017.

HILDEBRANDT, A. K. et al. Efficient computation of root mean square deviations under rigid transformations. Journal of Computational Chemistry, v. 35, p. 765-771, 2014.

HUA T. et al. Crystal structure of the human cannabinoid receptor CB1. Cell, v. 167, p. 750-762, 2016.

MAIOROV, V. N.; CRIPPEN, G. M. Significance of root-mean-square deviation in comparing three-dimensional structures of globular proteins. Journal of Molecular Biology, v. 235, p. 625-634, 1994.

MORGAN, C. A.; HURLEY, T. D. Characterization of two distinct structural classes of selective aldehyde dehydrogenase 1A1 inhibitors. Journal of Medicinal Chemistry, v. 58, p. 1964-1975, 2015.

PERTWEE, R. G. The diverse CB1 and CB2 receptor pharmacology of three plant cannabinoids: Δ9-tetrahydrocannabinol, cannabidiol and Δ9 -tetrahy-drocannabivarin. British Journal of Pharmacology, v. 153, p. 199-215, 2008.

RAMACHANDRAN, G. N.; RAMAKRISHNAN, C.; SASISEKHARAN, V. Stereochemistry of polypeptide chain configurations. Journal of Molecular Biology, v. 7, p. 95-99, 1963.

SANTOS, P. S. et al. Efeitos do ácido lipóico nas concentrações de glutamato e taurina no hipocampo de ratos após convulsões induzidas por pilocarpina. Arq. Neuro-Psiquiatr. [online]. 2011, vol.69, n.2b, pp.360-364.

MUNRO et al. Nature 365:61-65, 1993.

RINALDI-CARMONA M. et al. J. Pharmacol. Exp. Ther. 278:871-878, 1996.

Gupta A, Heimann AS, Gomes I, Devi LA. Antibodies against G-protein coupled receptors: novel uses in screening and drug development. Comb. Chem High Throughput Screen. 2008, 11(6)463-467.

Langmead CJ1, Watson J, Reavill C. Muscarinic acetylcholine receptors as CNS drug targets. Pharmacol Ther. 2008 Feb;117(2):232-43. Epub 2007 Dec 20.

Bomar MG & Galande AK. Modulation of the cannabinoid receptors by hemopressin peptides. Life Sci. 2013 92(8-9): 520-524.

Dvorácskó S, Tömböly C, Berkecz R, Keresztes A. Investigation of receptor binding and functional characteristics of hemopressin(1-7). Neuropeptides 2016, 58:15-22.

Gomes I, Grushko JS, Golebiewska U, Hoogendoorn S, Gupta A, Heimann AS. Ferro ES, Scarlata S, Fricker LD, and Devi LA. Novel endogenous peptide agonists of cannabinoid receptors. FASEB J. 2009 Sep; 23(9): 3020-3029.

Gelman JS, Sironi J, Castro LM, Ferro, ES, and Fricker LD. Hemopressins and other hemoglobin-derived peptides in mouse brain: Comparison between brain, blood, and heart peptidome and regulation in Cpefat/fat mice. J Neurochem. 2010 May; 113(4): 871-880.

Bauer M, Chicca A, Tamborrini M, Eisen D, Lerner R, Lutz B, Poetz O, Pluschke G, Gertsch J. Identification and Quantification of a New Family of Peptide Endocannabinoids (Pepcans) Showing Negative Allosteric Modulation at CB 1 Receptors. The Journal of Biological Chemistry Vol. 287, No. 44, pp. 36944-36967, October 26, 2012.

Reichwein JF & Liskamp RMJ. Site-specific N-alkylation of peptides on the solid phase. Tetrahedron Letters,

Volume 39, Issue 10, 5 March 1998, Pages 1243-1246.

Szlavicz E, Perera PS, Tomboly C, Helyes Z, Zador F, Benyhe S, Borsodi A, Bojnik E. Further Characterization of Hemopressin Peptide Fragments in the Opioid and Cannabinoid Systems. Anesth Analg. 2015 Dec;121(6):1488-94. doi: 10.1213/ANE.0000000000000964. PubMed PMID: 26465932.

Special Periodic Reports, Amino Acids, Peptides and Proteins: Volume 42, Royal Society of Chemistry, 2013.

From the reviewed literature, no document was found anticipating or suggesting the teachings of the present invention, let alone their surprising technical effects, so that the solution proposed here, in the eyes of the inventors, has novelty and inventive step over the state of the art.

## Summary of the Invention

The present invention addresses a number of known problems of the prior art, for example, to provide: a peptide compound which is more stable and easier to handle than hemopressin; a pharmaceutical composition for modulating metabolic functions and/or for improved analgesic effect over compositions containing hemopressin; an immunomodulatory pharmaceutical composition; a neuromodulatory, neuroprotective pharmaceutical composition for the curative or prophylactic treatment of convulsions and/or multiple sclerosis; a molecular entity which provides these and/or other technical effects without the disadvantages arising from the use of hemopressin such as instability, formation of aggregates and/or low therapeutic effect and without the inconveniences arising from the use of cannabinoid substances, such as prostration and nasal bleeding, among others.

It is a further object of the invention to provide a peptidic compound having high stability at extremes of temperature and/or ease of handling, being particularly useful in pharmaceutical preparations and medicaments. The peptidic compound of the invention, among others, provides advantages in the administration, bioavailability and/or therapeutic action in an animal when compared to other peptides, such as hemopressin and known variants, usually of larger size (9 to 15 aminoacids). The peptidic compound of the invention also provides oral administration to a mammalian animal and provides greater therapeutic effect than hemopressin, in addition to providing previously unknown uses and surprising in various aspects.

It is a further object of the invention to provide a compound substitute to cannabidiol and which provides advantages in the production, handling, use and safety and can replace it in all or almost all the applications already described therefor.

It is another object of the present invention to use the peptidic compound of the invention for the preparation of an improved pharmaceutical composition for modulating the metabolic functions of a mammal.

Another object of the present invention is the use of the peptidic compound of the invention for the preparation of an improved analgesic pharmaceutical composition.

It is another object of the present invention the use the peptidic compound of the invention for the preparation of a neuromodulator and/or neuroprotective medicament in a mammal.

It is another object of the present invention to use of the peptidic compound of the invention for the preparation of a medicament for the curative or prophylactic treatment of seizures in a mammal. In addition, administration of the peptidic compound of the invention to an animal provides important and surprising technical advantages, including superior anticonvulsant activity over cannabidiol and hemopressin, the use of which (hemopressin) as anticonvulsant being the subject matter of co-pending patent applications of the same inventors.

It is another object of the present invention the use of the peptidic compound of the invention for the preparation of a medicament for the curative or prophylactic treatment of multiple sclerosis in a mammal.

Other objects of the invention are therapeutic methods for: curative or prophylactic treatment of metabolic disorders, pain, seizures, multiple sclerosis, as well as for neuromodulation and/or neuroprotection.

Another object of the present invention is an improved pharmaceutical composition for modulating the metabolic functions of a mammal.

Another object of the present invention is an improved analgesic pharmaceutical composition.

Another object of the present invention is a neuromodulatory and/or neuroprotective pharmaceutical composition in a mammal.

Another object of the present invention is an improved pharmaceutical composition for the curative or prophylactic treatment of seizures in a mammal.

Another object of the present invention is a pharmaceutical composition for the curative or prophylactic treatment of multiple sclerosis in a mammal.

The compound of the invention is a peptidic compound of formula:
NFKF, NFKL, its salts, and/or combinations thereof.

The compound of the invention is synthetic and distinct from known natural forms and is useful for preparing a curative or prophylactic medicament for a mammal.

**The** pharmaceutical composition of the invention comprises the peptidic compound of the invention and a

pharmaceutically acceptable vehicle, and may be in the form of a tablet, gel, oral liquid or syrup, capsule, suppository, injectable solution or inhalable or adhesive forms, optionally comprising other active ingredients.

## Brief Description of the Figures

The following detailed description and the accompanying figures illustrate the main features and embodiments of the present invention, set forth in greater detail to provide further support to the person skilled in the art, and so that she/he may understand and reproduce the inventive concept of the invention in any of its embodiments. Also, some of the following examples, aspects and / or embodiments are not according to the invention and are present for illustration purposes only.

Figure 1 shows the results of comparative stability tests of the compound of the invention in the NFKF embodiment vs. Hp (PVNFKFLSH). The data on the graph shows the results of the concentration measurement of each of these actives by HPLC, after freezing for 24 hours or separately after heating at 100°C for 10 minutes (n = 2). In the vertical axis the area of the HPLC peak is shown. In A) is shown the Hp (control); in B) Hp after freezing for 24 hours; in C) Hp heated to 100°C; in D) the NFKF (control); in E) NFKF after freezing for 24 hours; in F) NFKF heated to 100°C. Statistical significance data are also shown, the asterisks indicating: (*) P <0.05 vs Control (***) P <0.005 vs Control; (****) P <0.0001 vs Control.

Figure 2 shows the test results of the compound of the invention in the NFKF embodiment compared to the hemopressin test results, both in the pilocarpine model. The % of time in relation to the control for the occurrence of the first salivation is presented with the administration of the following treatment doses: In A) the control (saline); in B) hemopressin (Hp or PVNFKFLSH, 0.551334 μmol/kg); in C) hemopressin (0.91889 μmol/kg); in D) the compound of the invention in the NFKF embodiment (0.540882 μmol/kg); in E) NFKF (0.901469 μmol/kg); in F) the compound PEP-19 (DIIADDEPLT, 0.908117 μmol/kg). The asterisks indicate the statistical significance: (*) P <0.05 vs Control; (**) P <0.01 vs Control; the + sign indicates P <0.05 vs Hp 0.91889 μmol/kg.

Figure 3 shows the test results of the compound of the invention in the NFKF embodiment as an anticonvulsant compared to the results of hemopressin tests as an anticonvulsant, both in the pilocarpine model. The percentage of time (relative to the control) for the first seizure is presented with the administration of the following treatment doses: in A) the control (saline); in B) hemopressin (Hp or PVNFKFLSH, 0.551334 μmol/kg); in C) hemopressin (0.91889 μmol/kg); in D) the compound of the invention in the NFKF embodiment (0.540882 μmol/kg); in E) NFKF (0.901469 μmol/kg); in F) the compound PEP-19 (DIIADDEPLT, 0.908117 μmol/kg). The asterisks indicate the statistical significance: (*) P <0.05 vs Control; (**) P <0.01 vs Control; the + sign indicates P <0.05 vs Hp 0.91889 μmol/kg.

Figure 4 shows the test results of the compound of the invention in the NFKF embodiment in the pilocarpine model, being indicated the time for occurring first salivation with the administration of: A) control; B) cannabidiol (30mg/kg); or C) NFKF (500μg/kg). The vertical axis shows the time in minutes. The data between control and other test compounds does not present statistical significance among themselves under the conditions tested.

Figure 5 shows the test results of the compound of the invention in the NFKF embodiment as an anticonvulsant in the pilocarpine model, being indicated the time for occurring the first convulsion with the administration of: A); control B) cannabidiol (30mg/kg); or C) NFKF (500μg/ g). The vertical axis shows the time in minutes. The asterisks indicate the statistical significance: (**) P <0.02 vs Control; (***) P <0.002 vs Control.

Figure 6 shows the results of neuroprotection/survival tests with the compound of the invention in the NFKF embodiment in the pilocarpine model, the survival/death profile of the animals being indicated by the administration of NFKF. In the vertical axis the number of survivors is shown. The horizontal axis shows the time in minutes. It is shown: in A) the survival profile of the animals administered with the control (saline only; in B) the survival profile of the animals administered with cannabidiol 30 mg/kg is shown; in C) the survival profile of animals given NFKF 500μg/kg; in D) all profiles are shown in a single graph. It is interesting to note that in the group treated with the neuromodulator NFKF 500 μg/kg only two animals died. The remaining animals in the NFKF group, for a total of 3, remained alive for more than a week, while practically all the others died in less than 30 minutes. Consequently, the mean survival time for this group is significantly different and much higher than the others. In addition, when comparing the NFKF group (500 μg/kg) with the group administered with cannabidiol (30 mg/kg), the survival rate is three times higher in the first group, that is, in the NFKF 500 μg/kg group, even using a 60-fold lower relative concentration of this compound.

Figure 7 shows the results of neuroprotection tests with different embodiments of the invention compound (NFKF,

NFKL) and the tripeptide NFK, all in 600 μg/kg, previously administered to groups of animals (n = 5 in each group) and subsequently administration of pilocarpine, showing the survival/death profiles of the animals after 24 hours in the model tested. The vertical axis shows the number of dead animals. The control is the administration of saline prior to the administration of pilocarpine.

Figure 8 shows the test results of the compounds of the invention NFKF, NFKL, as well as the tripeptides NFK, FKF, FKL and the dipeptides NF, FK, KF and KL in the pilocarpine model, indicating the time (min) for the occurrence of the first salivation after the administration of control or the test compounds. * P <0.05 vs Control. The results reflect the mean results of seven animals in the control group and six animals in the group treated with NFK.

Figure 9 shows the test results of the compounds of the invention NFKF, NFKL, as well as the tripeptides NFK, FKF, FKL and the dipeptides NF, FK, KF and KL in the pilocarpine model, indicating the time (min) for the occurrence of the first signal after administration of control or test compounds.

Figure 10 shows the test results of the compounds of the invention NFKF, NFKL, as well as the tripeptides NFK, FKF, FKL and the dipeptides NF, FK, KF and KL in the pilocarpine model, indicating the time for the occurrence of seizures after administration of control or test compounds. * P <0.05 vs control; ** P <0.001 vs control.

Figure 11 shows the test results of the compounds of the invention NFKF, NFKL, as well as the tripeptides NFK, FKF, FKL and the dipeptides NF, FK, KF and KL in the pilocarpine model, indicating the time (min) for the occurrence of death after administration of control or test compounds. * P <0.05 vs control.

Figure 12 shows results of the binding assays of various compounds to the CB1 receptor in anti-CB1 antibodies sensitive to activation/conformational changes of receptor. Treatment with 1 μM. The values in % of binding to the CB1 receptor in relation to control are indicated. * P <0.05 vs control.

Figure 13 presents an overview of the three-dimensional structure of a GPCR, in this case, the cannabinoid receptor of subtype 1. Reference is made to the seven transmembrane helices (I-VII), the intracellular loops (ICL1 and ICL2) and the extracellular loops (ECL2 and ECL3).

Figure 14 shows the overlap of AM6538 structure at the crystallographic structure (PDB 5TGZ), and the result obtained after validation by the Goldscore function (when the figure appears colored, the crystal structure is purple and the result of the Goldscore function in blue/cyan, although for the purposes of this application such colors are irrelevant).

Figure 15 shows the major interactions observed for AM6538 at the CB1 receptor binding site.

Figure 16 shows the main interactions observed for rimonabant at the CB1 receptor binding site.

Figure 17 shows the major interactions observed for cannabidiol at the CB1 receptor binding site.

Figure 18 shows the major interactions observed for the peptidic compound of the invention in the NFKF embodiment at the CB1 receptor binding site.

Figure 19 shows the results of the process of obtaining CB2 receptor structure. In A) the three-dimensional structure of the obtained CB2 receptor is shown; in B) the Ramachandran Graph for the obtained human CB2 model is shown.

Figure 20 shows the major interactions between the CB2 receptor and the AM6538 ligand.

Figure 21 shows the major interactions between the CB2 receptor and rimonabant.

Figure 22 shows the major interactions between the CB2 receptor and cannabidiol.

Figure 23 shows the major interactions between the CB2 receptor and the compound of the invention in the NFKF embodiment.

Figure 24 shows the pain threshold results, represented as force in grams, applied to the back of the right paw of rats. When animals react by kicking, the force in grams required to induce this response is the pain threshold. The vertical

axis shows the pressure applied at the leg. Antinoniceptive activity was expressed as the increase in pain threshold compared to control animals. The compounds of the invention were administered orally to animals at doses of 0.5 to 0.25 mg/kg and saline was used as a control. * P <0.05 vs control.

Figure 25 shows the test results of the compound of the invention in the NFKF embodiment for the control of multiple sclerosis. The vertical axis shows the clinical score. The horizontal axis shows the number of days after immunization. A) shows the Clinical Score curve of the Wild Type (WT) mouse, that is, normal, submitted to EAE induction with MOG; B) shows the Clinical Score curve of the knock out mouse of the endopeptidase gene 24.15, that is, transgenic and prone to Multiple Sclerosis symptoms, in the EAE model, submitted to MOG induction; C) shows the Clinical Score curve of the knock out mouse of the endopeptidase gene 24.15, that is, transgenic and prone to Multiple Sclerosis symptoms, in the EAE model, submitted to MOG induction and the neuroprotection obtained with NFKF * P <0.05 24.15 KO + NFKF vs WT; + P <0.05 24.15 KO vs WT.

## Detailed Description of the Invention

The inventive concept underlying the several objects of the invention is a peptidic compound of formula: NFKF, NFKL, its salts, and/or combinations thereof.

The compound of the invention is synthetic and distinct from known natural forms and is useful for preparing a curative or prophylactic medicament for a mammal.

Said peptidic compound has surprisingly high stability at extremes of temperature and ease of handling, being particularly useful in pharmaceutical preparations and medicaments. The peptidic compound of the invention, among others, provides advantages in the administration, bioavailability and/or therapeutic action in an animal when compared to other peptides, such as, for example, hemopressin and known variants, usually of larger size (9 to 23 amino acids). The peptidic compound of the invention also provides for oral administration to a mammalian animal and provides a greater therapeutic effect than those known for hemopressin and may advantageously replace hemopressin in all or almost all applications already described therefor.

The compound of the invention is also a synthetic intermediate in the preparation of compounds of pharmaceutical interest which comprise the peptidic compound of the invention and include chemical modifications, substitutions, inclusion of other functional groups.

The compound of the invention is an advantageous substitute for cannabidiol and provides advantages in the production, handling, use and safety and is useful for replacing cannabidiol in all or almost all applications already described therefor.

The compound of the invention is useful in an improved pharmaceutical composition for modulating the metabolic functions of a mammal.

The compound of the invention is useful in an improved analgesic pharmaceutical composition.

The compound of the invention is useful in a pharmaceutical composition for the curative or prophylactic treatment of seizures in a mammal. Administration of the peptidic compound of the invention to an animal provides important and surprising technical advantages, including superior anticonvulsant activity over cannabidiol and hemopressin, the use of the latter as an anticonvulsant being the subject of co-pending patent application PCT/BR2017/050313, of the same inventors.

The compound of the invention is useful in a neuromodulatory and/or neuroprotective pharmaceutical composition in a mammal. The test results presented in the present patent application show that the composition of the invention modulates action of neurons and is also neuroprotective, anticonvulsive and is useful in the treatment of multiple sclerosis.

For purposes of the present invention the following definitions are used.

### Product of pharmaceutical interest

In the context of the present application, "compound of pharmaceutical interest" means any molecular entity comprising the compound described as inventive concept common to the present application, including also molecular entities obtained by chemical modification/derivatization of the same, or with the inclusion of other functional groups, linear or branched side chains, alteration of hydrophilicity or hydrophobicity, among others, provided that they comprise as nucleus the entity NFKF, NFKL, its salts, and/or combinations thereof as defined above, except for the natural and already known entities.

### Pharmaceutical composition

In the context of the present patent application, "pharmaceutical composition" is to be understood as any and all compositions containing an active principle, for prophylactic, palliative and/or curative purposes, acting in a manner to

maintain and/or restore the homeostasis, and may be administered orally, topically, parenterally, enterally and/or intrathecally.

Pharmaceutically acceptable formulation

In the context of the present application a "pharmaceutically acceptable formulation" is meant as a formulation containing pharmaceutically acceptable excipients and carriers well known to those skilled in the art, such as the development of convenient doses and treatments for use in particular compositions which can be described in a number of treatment regimens, including oral, parenteral, intravenous, intranasal, intravitreal and intramuscular, intracerebral, intracerebroventricular and intraocular treatment and administration and/or formulation.

Modified peptide

In the context of the present application, "modified peptide" is to be understood as a non-naturally occurring, artificially modified or synthesized peptide, including halides, cyclized, amidated, alkylated, alkoxylated, hydroxylated, PEGylated forms, forms with other functional groups on any amino acid, or salt forms thereof, as well as on an amino acid or peptide, including the non-natural, such as d-amino acid forms. The peptidic compound may be pegylated using techniques known to those skilled in the art, such as, for example, pegylation with reagents containing the succinimidyl group, which preferentially react with primary amines present in the N-terminal region of the peptide. The peptidic compound of the invention may be alkylated at any amino acid using techniques known to those skilled in the art, including, for example, the Mitsunobu reaction described in Reichwein & Liskamp (Reichwein JF & Liskamp RMJ, Site-specific N-alkylation of peptides on the solid phase, Tetrahedron Letters, Volume 39, Issue 10, 5 March 1998, Pages 1243-1246). Said article describes the introduction of any alkyl group into a specific amide function of a peptide. The peptidic compound of the invention may be alkoxylated, substituted with halogens, hydroxy or other functional groups on any amino acid using techniques known to those skilled in the art, including, for example, those described in the book Special Periodic Reports, Amino Acids, Peptides and Proteins: Volume 42, Royal Society of Chemistry, 2013. The peptidic compound of the invention may be modified with other molecular species useful in diagnostic and/or therapeutic applications, such as Biotin, using techniques known to those skilled in the art.

Cyclic or circular peptide

In the context of the present application, "cyclic, cyclized or circular peptide" is to be understood as a peptide which has a covalent bond between the two ends of a linear peptide molecule by any method known in the art, particularly by the activity of enzymes. The cyclic peptide can be used instead of the linear peptide because it is more difficult to be degraded, since its ends or zones of attack by hydrolyzing enzymes are not as exposed as in a linear peptide.

Agonist

In the context of the present application, "agonist" is to be understood as a drug, drug, hormone, neurotransmitter or other signaling molecule which forms a complex with a receptor site, thereby triggering an active response of a cell.

Inverse agonist/antagonist

In the context of the present application, "inverse agonist or antagonist" shall be understood as agent(s) (for example, drugs, drugs, hormones or enzymes) which bind(s) to agonist receptors and produce(s) pharmacological effects opposite to those of the agonists, such that the action of one partially or totally inhibits the effect of the other. Particularly, a compound is an inverse agonist when it acts in the presence of an agonist, but reducing its activity; an antagonist is a compound that will totally block the activity of the agonist.

Equivalent Dose in Humans

In the present invention, the concept of "equivalent dose in humans" is the dose at which, in humans, the same magnitude of effects is expected in animals at a given dose, as set forth in "Guidance for Industry Estimating the Maximum Safe Starting Dose in Initial Clinical Trials for Therapeutics in Adult Healthy Volunteers" published by the US Department of Health and Human Services Food and Drug Administration Center for Drug Evaluation and Research (CDER), July 2005 Pharmacology and Toxicology. In said guide, the conversion of the observed dose in animals (mg/kg) to Equivalent Dose in Humans (mg/kg) entails dividing the result obtained in rats by 6.2 and the result obtained in mice by 12.3. These values are applicable to a human of 60 kg standard weight. For other species or for weights outside the standard weight ranges, the

Dose Equivalent in Humans (DEH) can be calculated by the formula: DEH = dose in animal in mg/kg x (animal weight in kg/human weight in kg) $^{0.33}$. This Guidance considers a safety range of 10 times the concentration limits tested to be adequate.

Ligand of CB receptor

In the context of the present patent application, a "ligand of CB receptor" is to be understood as a compound or molecule that interacts with the CB system and/or with CB1 or CB2 receptors.

Modulating the CB receptor function or the cannabinoid system

In the context of the present patent application, it is meant to "modulate the CB receptor function" as an interaction that results in the alteration of the biochemical activity of the CB receptor, particularly CB1 or CB2. It is understood that the change is positive when an antagonist or inverse agonist effect occurs at CB receptors and that the change is negative when an agonist effect occurs at CB receptors. The results of the tests presented in the present patent application indicate that the compound of the invention interacts with and/or modulates the CB1 receptor and/or the CB2 receptor, probably as an allosteric modulator of CB1 and/or CB2 receptor. Thus the compound of the invention is useful for modulating the cannabinoid system, either by modulating the CB1 receptor, or the CB2 receptor, of both concomitantly, by modulating the binding or action of other substances interacting in the cannabinoid system, by modulating proteases or peptidases which lead to the generation or degradation of active peptides in the cannabinoid system, or by improving the natural system by the protection of natural molecules with the compounds of the invention, that is, the compound of the invention could also lead the natural system to a protective action independently of the binding to said receptors, or combinations of said effects.

Modulating the function of muscarinic receptors

In the context of the present application, the term "modulating the function of muscarinic receptors " should be understood as an interaction which leads to alterations in muscarinic acetylcholine receptors (mAChRs), which play an important role in cognitive functions, such as learning and memory, control of dopamine release, modulation of locomotor activity, its modulation being also useful in the control of Alzheimer's disease and/or control of dependency or addiction to abuse drugs. It is understood that the change is positive when an antagonist or inverse agonist effect occurs at muscarinic receptors and that the change is negative when an agonist effect occurs at muscarinic receptors. The tests presented in the present patent application suggest that the compound of the invention interacts with and/or modulates muscarinic receptors.

Modulation of metabolic functions

In the context of the present application, this term is understood to include modulation of: energy and/or lipid metabolism; arterial hypertension, regulation of intestinal motility; immune system; balance of the calcium cycle, conditions associated with the thyroid gland, peripheral organs and tissues, including reproductive organs, adipose tissue, liver, muscles and gastrointestinal tract, being useful in the treatment of obesity, diabetes, diseases or immune/inflammatory disorders, osteopenia, osteoporosis, cancer. In this context, the peptidic compounds of the invention are also useful in pharmaceutical compositions for the treatment of metabolic disorders comprising preventing overweight; regulation of appetite; induction of satiety; prevention of weight gain after successful weight loss; increased energy consumption; aesthetic weight reduction; or bulimia.

Neuromodulator

In the context of the present application the term "neuromodulator" or "neuromodulator" is intended to modulate neuronal/neurological function, including modulation of brain, cortex, hippocampus, amygdala, pituitary, hypothalamic activity; adrenal gland. Neuromodulation includes the beneficial modulation of neuroprotection against agents or conditions leading to pathophysiological processes. Neuroprotective agents or compounds are preferably used prior to (or during) the prodromal stage of disease, which often begins many years before the onset of symptoms. In the present invention, a neuromodulator is potentially useful in the curative or prophylactic treatment of a variety of neurological conditions or diseases, including essential tremor, migraine, pain, neuropathic pain, multiple sclerosis, amyotrophic lateral sclerosis, psychiatric disorders such as anxiety, schizophrenia or bipolar disorder, congenital disorders such as dementia, Alzheimer's, Parkinson's, autism and also potentially useful in modifying the pathophysiological processes involved in the occurrence of seizures and/or epilepsy, as well as in other clinical conditions related to disorders of neuronal excitability or

neuronal lesions due to ischemia, hypoxia or other harmful conditions.

Although in this patent application it is demonstrated that the compound of the invention binds and/or modulates cannabinoid receptors, the surprising pharmaceutical action of the invention may be linked to action on CB1 and/or CB2 and/or muscarinic receptors or possibly be linked to modulation of uptake of adenosine, GGPR55, PPARγ receptors, intracellular calcium level, modulation of opioid receptors that form heterodimers with cannabinoid receptors, or combinations thereof. Thus, any therapeutic indication related to these targets may benefit from the present invention.

The present invention as set out in the appended claims also refers, amongst others, to the following:
A pharmaceutical composition modulating metabolic functions in a mammal comprising a pharmaceutically acceptable carrier; and, as active principle, the compound described above.

Analgesic pharmaceutical composition in a mammal comprising a pharmaceutically acceptable carrier; and, as active principle, the compound described above.

A neuromodulatory and/or neuroprotective pharmaceutical composition comprising a pharmaceutically acceptable carrier; and, as active principle, the compound described above.

A pharmaceutical composition for the curative or prophylactic treatment of seizures in a mammal comprising a pharmaceutically acceptable carrier; and, as active principle, the compound described above.

Pharmaceutical composition for the curative or prophylactic treatment of Multiple Sclerosis in a mammal comprising a pharmaceutically acceptable carrier; and, as active principle, the compound described above.

Pharmaceutical composition as described above in the form of a tablet, tablet, gel, oral liquid or syrup, capsule, suppository, solution for injection, inhalable form or in adhesive.

**The** results of *in vitro* and *in silico* tests have shown that the compound of the invention provides advantages in the preparation of pharmaceutical compositions, in their stability, and in therapeutic effects.

The compound of the invention has proved to be much more stable than hemopressin, which in addition causes technical problems of fiber formation or fibrils, as well as its known variants.

The results of *in vivo* tests in mammals showed excellent therapeutic effect at low dosages, with no evidence of significant side effects

The peptidic compound of the invention has also been shown to be an appropriate synthetic intermediate for the preparation of other compounds useful in diagnostic and/or therapeutic applications. Tests conducted in the present invention show that the compound of the invention is suitably modified with biotin known to those skilled in the art as useful in diagnostic preparations or kits. The peptidic compound of the invention, when modified/protected by biotin, has also been shown to bind to the CB1 receptor in tests with anti-CB1 antibodies sensitive to the activation/conformational changes of the receptor. Another object of the invention is an in vitro method of diagnosing the presence, amount and/or localization of cannabinoid, opioid and/or muscarinic receptors, as well as evaluating the binding of other compounds to these receptors.

The neuromodulator/neuroprotective effect resulting from the administration of the compound of the invention in mammals is evidenced by the decrease and/or absence of symptoms, brain damage and death associated with the administration of substances known to be harmful, such as pilocarpine. Tests with the EAE/MOG model, the most commonly used model for replicating multiple sclerosis, also show that administration of the peptide compound of the invention to an animal substantially diminishes the clinical symptoms of multiple sclerosis, a disease essentially related to neuronal damage.

In one embodiment, the invention provides the use of said compound for the preparation of a neuromodulatory, neuroprotective medicament and/or for the curative or prophylactic treatment of seizures in a mammal. Administration, to an animal, of the compound of the invention provides neuromodulatory, neuroprotective, anticonvulsive and/or symptomatic multiple sclerosis inhibiting activity; makes oral administration feasible; does not have or entails the drawbacks arising from the production, storage, transport and use of cannabinoid substances, in addition to providing additional advantages in the preparation of therapeutic products for mammals in their administration and/or effects. In addition, administration of the compound of the invention to an animal provides important and surprising technical advantages, including superior anticonvulsant activity with respect to hemopressin, the use of which as an anticonvulsant is the subject of the co-pending co-pending application of the same inventors.

The present invention describes the use of a compound for the preparation of pharmaceutical compositions useful for a wide variety of medical conditions, including those related to the central nervous system. Surprisingly, although the active compound of the invention is peptidic or predominantly peptidic, oral administration has provided brain effects in animals.

In one embodiment, in vivo tests with the composition of the invention have shown surprising results as to its neuroprotective activity. The composition of the invention, when administered previously to animals, provided surprising, potent and long protection against damage resulting from the subsequent administration of substances known to be harmful to neurons. Accordingly, the neuroprotection provided by the compound of the invention is particularly useful as a therapeutic alternative for various medical conditions, including those related to disorders of neuronal excitability, such as seizures.

In various embodiments, in vivo tests with the compound of the invention demonstrated surprising results regarding its anticonvulsant activity. The compound of the invention, when used as an anticonvulsant, additionally provides the advantage of being a good candidate to substitute for the cannabinoid compounds known to act as anticonvulsants, such as cannabidiol. Cannabidiol, despite its proven effects as an anticonvulsant, has been facing regulatory problems due to its origin, the *Cannabis sativa* plant. The present invention provides an additional therapeutic approach for patients suffering from seizures and having difficulty in obtaining drugs, being based on a peptide, ie, does not use derivatives of *Cannabis sativa.* In addition, the results showed that the compound of the invention, when used as an anticonvulsant, provides other surprising technical advantages in use, including greater therapeutic effect, oral use, lower dosage, less occurrence of side effects such as prostration and nasal bleeding, among others technical advantages.

The molecular mechanism of how cannabidiol (CBD) ceases seizures is not yet fully understood. It is known that CBD: inhibits adenosine reuptake; is a 5-HT 1A agonist; is an antagonist of GPR55 (CB3); is a PPAR$\gamma$ receptor agonist; increases intracellular Ca2+, in addition to interacting with CB1 and CB2. Hemopressin, on the other hand, has a predominant action via pERK1/2 and AKT. It is therefore surprising and at a difficult time to explain why both the peptidic compound of the invention and the CBD have anti-convulsive action.

In the experimental convulsion model tests, administration of the pharmaceutical composition of the invention to mammals resulted in excellent therapeutic effect at low dosages when compared to dosages of a reference compound, cannabidiol. In addition, test results have shown that the composition of the invention provides surprising technical advantages in use, including greater therapeutic effect, viability of oral use, lack of use of carrier oil (which in many cases causes side effects), lower dosage and lower occurrence of side effects such as prostration and nasal bleeding, among others.

*In vivo* comparative tests demonstrate that the compound of the invention has superior activity and/or requires less dosage than hemopressin, the use of which as an anticonvulsant is the subject of the co-pending patent application of the same inventors.

The pharmaceutical composition of the invention is also useful for the treatment of diseases associated with modulation of the activity of the cannabinoid system, cannabinoid (CB) and/or muscarinic receptors - with several technical advantages and without known undesirable effects of the available congeners in the state of the art.

Additionally, as will be demonstrated in the following examples, the use of the compound of the invention in the preparation of a neuromodulatory, neuroprotective and/or anticonvulsant drug provides for the delivery of a medicament orally administrable to a mammal. Test results revealed significant brain action, suggesting that administration of the compound of the invention allows the active element to cross the blood brain barrier. Thus, the results show/support the use of the compound of the invention regardless of whether the compound of the invention is the active which acts directly on the target, ie, does not degrade during oral ingestion, or the compound is a precursor which, upon modification chemistry, acts on the target - in this case, being characterized as a pro-drug. This feature of providing important effects even by oral administration is particularly desirable, since the natural enzymes of a mammal in general degrade peptides and proteins while in the digestive tract and rarely a drug with a peptidic active is shown to be viable. However, surprisingly, the compound of the invention - even when administered orally - provides strong therapeutic action, in this case even more surprisingly, acting in the brain.

Thus, regardless of the mechanism of action, which is not the subject of the present application, the oral administration of the pharmaceutical composition of the invention has provided important neuromodulatory, neuroprotective, anticonvulsive, pain-modulating and symptomatic multiple sclerosis and even avoiding deaths, clearly shows the surprising magnitude and relevance of solved technical problems.

The pharmaceutical composition of the invention comprises the compound described above and also a pharmaceutically acceptable carrier, optionally also comprising other pharmaceutically acceptable actives and/or salts thereof. The pharmaceutical composition of the invention may be administered in the form of a tablet, gel, capsule, oral liquid or syrup, a suppository, an injectable solution or other suitable forms of administration for pharmaceutical and medical purposes.

The following examples are only intended to exemplify some of the various ways of practicing the invention, however, without limiting the scope thereof.

Comparative tests showed that the compound of the invention showed superior *in vitro* stability at temperature extremes when compared to hemopressin.

In some examples, the neuromodulatory/neuroprotective/anticonvulsive effects of the composition of the invention have been evaluated *in vivo* by oral administration to animals. The pharmaceutical composition of the invention was administered to mammals (*Mus musculus* or mouse) with an oral dose of treatment with different embodiments of the compound of the invention, compared to other compounds or to saline control. In these experiments, the test compounds were administered orally 10 minutes prior to (intraperitoneal) administration of pilocarpine. Pilocarpine hydrochloride (320 mg/kg, Merck), dissolved in 0.9% sterile saline, was given intraperitoneally for induction of SE (status epileticus) (Turski et al., 1983). In the Turski model, the neurotoxic effects begin about 15-25 minutes after the injection of Pilo, with the occurrence of motor and limbic seizures, the animals evolving to a state of continuous (clonic) seizures that characterize

SE Sanabria and Cavalheiro, 2000).

Comparative tests also showed that the compound of the invention showed superior in vivo therapeutic activity when compared to hemopressin, the use of which as an anticonvulsant is the subject of co-pending patent application PCT/BR/2017050313, the same inventors.

The compound of the invention, being a ligand/modulator of an important GPCR (cannabinoid receptor), is useful in modulating GPCR receptor activity under pathological conditions, as well as modulating the target GPCR and also as a carrier of other targeted therapeutic molecular entities to cells expressing GPCRs that dimerize with cannabinoid receptors. The compound of the invention is also useful in therapies combined with antibodies, especially monoclonal antibodies, selective for the activated/modulated conformation of the receptor. Such therapies provide the advantage of conferring high specificity, potentially reducing also the dose of administration.

## Examples

### Example 1. Stability tests under extreme conditions - Superiority of the compound of the invention in relation to Hemopressin

In this embodiment, the stability of the compound of the invention in the NFKF embodiment was compared to that of hemopressin (Hp, PVNFKFLSH) under extreme conditions. Hp is known to have the problem of fibril formation, as well as variants thereof which have a greater number of amino acids. Samples of NFKF and Hp were subjected to two separate tests, that of stability by freezing for 24 hours and heating at 100°C for 10 minutes.

The results of figure 1 show that, by freezing for 24h, a significant part of Hp is lost or degraded (from 140 to 97, i.e. approximately 31%), as evidenced by measurements by HPLC (analytical column of 2.1mm, with gradient running of 10-60% B. Solvent A is water/0.1% TFA and solvent B is acetonitrile/0.075% TFA). The compound of the invention in the NFKF embodiment, on the other hand, remained much more stable and suffered substantially less degradation (from 120 to 100, ie, 16.7%). The results of Figure 1 also show that upon heating at 100°C for 10 minutes, even more significant part of the Hp is lost or degraded (from 140 to 50, ie approximately 64.3%), as evidenced by measurements by HPLC. On the other hand, the compound of the invention in the NFKF embodiment remained stable and did not suffer statistically significant degradation.

Together, these data show that the compound of the invention provides much greater stability and lower degradation under extreme temperature conditions, which favors it in the manipulation, galenics, pharmaceutical preparations and stability of the pharmaceutical both in the post-acquisition phase of the active principle, and in the industrial production of medicines and, not least, in the transport logistics chain. The data suggest that the shelf life of the pharmaceutical product containing the compound of the invention should be greater than the congeners containing Hp or other actives with instability problems.

### Example 2. Use of compound NFKF for the preparation of pharmaceutical composition

In this embodiment, the compound is NFKF, which has been synthesized by chemical synthesis. Said peptide has been used in the preparation of an oral liquid pharmaceutical composition comprising between $2.7 \times 10^{-4}$ Molar of said peptide and a pharmaceutically acceptable carrier. In this embodiment, said carrier is saline, the pharmaceutical composition being a solution for oral use. Said composition was used for *in vivo* oral administration to mammals according to examples 3-8 below.

In other embodiments, the pharmaceutical composition is in the form of a tablet, gel, oral liquid or syrup, capsule, suppository, injectable solution or inhalable or adhesive forms, optionally comprising other active principles.

### Example 3. Comparative pharmaceutical composition comprising the compound NFKF with the pharmaceutical composition comprising Hp - results of *in vivo* tests

In this example, the effects of the composition of the invention with respect to the pharmaceutical composition containing Hemopressin (Hp or PVNFKFLSH), the use of which as anticonvulsant is co-pending patent application PCT/BR2017/050313, of the same inventors, were compared.

Figure 2 shows the test results of the compound of the invention NFKF compared to the hemopressin test results, both in the pilocarpine model. The percentages of time in relation to the control for the occurrence of the first salivation are presented with the administration of the following treatment doses: control (saline); hemopressin (Hp or PVNFKFLSH, 0.551334 μmol/kg); hemopressin (0.91889 μmol/kg); the peptide of the invention NFKF (0.540882 μmol/kg); NFKF (0.901469 μmol/kg); or PEP-19 (DIIADDEPLT, 0.908117 μmol/kg). The asterisks indicate the statistical significance: (*) $P < 0.05$ vs Control; (**) $P < 0.01$ vs Control; the + sign indicates $P < 0.05$ vs Hp 0.91889 μmol/kg.

On the one hand, salivation induced by administration of pilocarpine is indicative of changes in muscarinic

receptors. Accordingly, the substantial change in the time profile for the occurrence of the first salivation observed with prior administration of the compound of the invention suggests modulation, either directly or indirectly, of muscarinic receptors.

In addition, the results of Figure 2 clearly show that oral administration of the compound of the present invention provides substantially higher salivation time modulating activity substantially greater than that observed with oral administration of Hp.

**Example 4. Comparative anticonvulsant pharmaceutical composition comprising the compound NFKF with the anticonvulsant pharmaceutical composition comprising Hp - results of *in vivo* tests**

In this example, the anticonvulsive effects of the composition of the invention were compared to the pharmaceutical composition containing Hemopressin (Hp or PVNFKFLSH), the use of which as an anticonvulsant is co-pending patent application PCT/BR2017/050313, the same inventors.

Figure 3 shows the test results of the compound of the invention NFKF as an anticonvulsant compared to the results of tests of hemopressin as an anticonvulsant, both in the pilocarpine model. The percentages of time (relative to the control) for the first seizure occur with administration of the following treatment doses: hemopressin (Hp or PVNFKFLSH, 0.551334 μmol/kg); hemopressin (0.91889 μmol/kg); the peptide of the invention NFKF (0.540882 μmol/kg); NFKF (0.901469 μmol/kg); PEP-19 (DIIADDEPLT, 0.908117 μmol/kg) and control (saline). The asterisks indicate: (*) P <0.05 vs Control; (**) P <0.01 vs Control; the + sign indicates P <0.05 vs Hp 0.91889 μmol/kg.

Furthermore, the results of Figure 3 clearly show that oral administration of the compound of the present invention provides activity modulating the occurrence of the first seizure substantially greater than that observed with oral administration of Hp. For example, the same effect is observed with half the dose of the compound of the invention when compared to Hp.

**Example 5. Anticonvulsive pharmaceutical composition comprising the compound NFKF - results of *in vivo* tests.**

In this embodiment, the anticonvulsive effect of the composition of the invention prepared according to example 2 was evaluated by prior administration of the inventive composition and subsequent administration of pilocarpine to animals. Figure 4 presents the results of the pilocarpine model, indicating the time for the first salivation with the administration of cannabidiol (30mg / kg) of the compound of the invention, NFKF tetrapeptide (500μg/kg).

Figure 5 presents the results of pilocarpine modeling, indicating the time for the first seizure to occur with the administration of cannabidiol (30mg/kg) and the peptide of the invention NFKF (500μg/kg). The asterisks indicate: (**) P <0.02 vs Control; (***) P <0.002 vs Control.

In addition to the results shown in Figures 4 and 5, important additional technical effects include observation of no occurrence of prostration or nasal bleeding in animals undergoing treatment with the compound of the invention NFKF, whereas in the group of animals treated with cannabidiol both prostration and the nasal bleeding was observed. These, therefore, are additional technical problems solved by the compound of the invention.

**Example 6. Neuromodulatory pharmaceutical composition comprising compound NFKF - results of *in vivo* tests**

In this embodiment, the neuromodulatory effect of the composition of the invention prepared according to Example 2 was evaluated by prior administration of the inventive composition and subsequent administration of pilocarpine to animals. Other test compounds were also evaluated as described below. Administration of pilocarpine leads to severe brain injury, neurotoxicity and usually culminates in the death of the animals. This substance was used in the experiments described below but its harmful neuronal/encephalic effects were inhibited by the prior administration of the composition of the present invention: the vast majority of the animals subjected to these experiments had no symptoms related to brain lesions and survived without apparent damage, in contrast to groups of animals treated with other known substances.

The composition of the invention prepared according to Example 2 was pre-administered to the animals. Figure 6 shows the results of neuroprotection tests with the compound of the invention on the NFKF embodiment in the pilocarpine model, the survival/death profile of the animals being indicated by the administration of NFKF. In A) the survival profile of the animals administered the control (saline only) is shown; In B) the survival profile of the animals administered cannabidiol 30 mg/kg is shown; In C) the survival profile of animals given NFKF 500μg/kg was shown; In D) all profiles are shown in a single graph. Interestingly, in the group treated with NFKF 500 μg/kg only two animals died. The remaining animals in the NFKF group, for a total of 3, were alive for more than a week, while practically all the others died in less than 30 minutes. Consequently, the mean survival time for this group is significantly different and much higher than the others. In

addition, when comparing the NFKF group (500 μg/kg) with the group administered cannabidiol (30 mg/kg), the survival rate is three times higher in the first group, that is, in the group administered NFKF 500 μg/kg, even using a 60-fold lower relative concentration of compound.

In addition to the results shown in Figure 6 above, important additional technical effects include observation of non-occurrence of prostration or nasal bleeding in animals undergoing NFKF treatment, while in the group of animals treated with cannabidiol both prostration and bleeding were observed. These, therefore, are additional technical problems solved by the compound of the invention.

**Example 7. Neuromodulatory pharmaceutical composition comprising compound NFKF or NFKL - results of *in vivo* tests**

In view of the surprising results obtained in Example 6, the peptides of the invention in the NFKF and NFKL embodiments were compared to each other and to NFK in the experiment described below, which shows that the harmful neuronal/encephalic effects of pilocarpine were inhibited by the prior administration of these embodiments of compounds of the present invention: some animals subjected to these experiments had no symptoms related to brain lesions and survived without apparent damage, in contrast to the group of animals treated with the control. The composition of the invention was prepared according to Example 2 with due concentration correction, for comparison of the effects of NFKF, NFKL and NFK, such compounds having been previously administered to the animal groups. Figure 7 shows the results of neuroprotection tests in the pilocarpine model, the survival/death profile of the animals being indicated by administration of these compounds of the invention and subsequently to pilocarpine. It is interesting to note that in the group treated with NFKL 600 μg/kg the results were even better than those obtained with NFKF, whereas the results with NFK were equivalent to those obtained with NFKF.

**Example 8. An anticonvulsant pharmaceutical composition comprising the compounds NFKF, NFKL - results of *in vivo* tests**

In this embodiment, the anticonvulsant effect of different embodiments of the compound of the invention NFKF and NFKL as well as the NF, FK, KF and KL dipeptides and the NFK, FKF and FKL tripeptides were evaluated in the pilocarpine model.

Table 1 shows the time data of occurrence of the first salivation in the model tested.

Table 1:

| | control | NFKF | NFKL | NFK | FKF | FKL | NF | FK | KF | KL |
|---|---|---|---|---|---|---|---|---|---|---|
| 1 | 0.6667 | 1.83 | 1.183 | 1.38 | 0.38 | 1.6 | 0.83 | 0.1833 | 1.38 | 0.95 |
| 2 | 3.2000 | 1.97 | 1.067 | 0.75 | 0.95 | 0.2 | 0.97 | 1.5 | 1.75 | 0.50 |
| 3 | 1.6333 | 2.73 | 1.25 | 0.42 | 0.50 | 0.633 | 1.73 | 0.95 | 1.42 | 1.50 |
| 4 | 2.6667 | 1.58 | 2.45 | 0.98 | 1.50 | 1.5 | 0.58 | 2.183 | 1.98 | |
| 5 | 0.8333 | 3.2 | 1.25 | | | 0.333 | 1.2 | | | |

Figure 8 presents the test results of the compounds of the invention NFKF and NFKL as well as the dipeptides NF, FK, KF, KL and the tripeptides NFK, FKF and FKL in the pilocarpine model, indicating the time for the occurrence of the first salivation after administration of control or the test compounds. It is noteworthy the result of the administration of NFK, which inhibited or delayed the occurrence of first salivation in the pilocarpine model. This result indicates the modulation of muscarinic receptor function, such as acetylcholine (mAChRs), which plays an important role in cognitive functions, such as learning and memory, control of dopamine release, modulation of locomotor activity. Modulation of these receptors is useful in the control of Alzheimer's disease and/or control of dependence or addiction of drugs of abuse, as well as in protection against dementia of old age.

Table 2 shows the time data of occurrence of the first signal in the model tested.

Table 2:

| | control | NFKF | NFKL | NFK | FKF | FKL | NF | FK | KF | KL |
|---|---|---|---|---|---|---|---|---|---|---|
| 1 | 2.67 | 8.43 | 9.317 | 6.28 | 5.3 | 5.67 | 5.67 | 6.5 | 5.8 | 8.5 |
| 2 | 10.2 | 10.23 | 10.17 | 8.92 | 6.35 | 9.5 | 4.99 | 7.33 | 6.33 | 7.33 |

(continued)

| | control | NFKF | NFKL | NFK | FKF | FKL | NF | FK | KF | KL |
|---|---|---|---|---|---|---|---|---|---|---|
| 3 | 10.5 | 13.53 | 3.83 | 4.83 | 8.33 | 8.5 | 5.5 | 7.4 | 6.5 | |
| 4 | 5.5 | 9.9 | 10.17 | 7.82 | 8.82 | 4.5 | 3.5 | 6.17 | 7.5 | |
| 5 | 9.65 | 12.33 | 10.25 | | | 9.17 | 6.5 | | | |

Figure 9 shows the test results of the compounds of the invention NFKF and NFKL, as well as the dipeptides NF, FK, KF and KL and the tripeptides NFK, FKF and FKL in the pilocarpine model, indicating the time for the occurrence of the first signal after control administration or the test compounds.

Table 3 shows the time data of occurrence of the first seizure in the model tested.

Table 3:

| | control | NFKF | NFKL | NFK | FKF | FKL | NF | FK | KF | KL |
|---|---|---|---|---|---|---|---|---|---|---|
| 1 | 7.9 | 15.17 | 18.4 | 11.33 | 6.28 | 5.3 | 5.67 | 5.67 | 6.5 | 5.8 |
| 2 | 10.5* | 19.95 | 15.33 | 9.67 | 8.92 | 6.35 | 9.5 | 4.99 | 7.33 | 6.33 |
| 3 | 8.38 | 15.18 | 30 | 8 | 3.83 | 4.83 | 8.33 | 8.5 | 5.5 | 7.4 |
| 4 | 9.55 | 16 | 27.3 | 9.93 | 7.82 | 8.82 | 4.5 | 3.5 | 6.17 | |
| 5 | 9.65 | 17.88 | 15.33 | 13.38 | 10.25 | | 9.17 | 6.5 | | |

Figure 10 shows the test results of the compounds of the invention NFKF and NFKL, as well as the dipeptides NF, FK, KF and KL and the tripeptides NFK, FKF and FKL in the pilocarpine model, indicating the time for the occurrence of the first seizure after control administration or the test compounds.

Table 4 shows the time data of death occurrence in the model tested.

Table 4:

| | contro l | NFK F | NFK L | NFK | FKF | FKL | NF | FK | KF | KL |
|---|---|---|---|---|---|---|---|---|---|---|
| 1 | 13.17 | 18.92 | 26.63 | 13.53 | 13.17 | 14.53 | 13.17 | 13.17 | 11.7 | 16.67 |
| 2 | 18.67 | 20 | 25.13 | 11.7 | 20.67 | 11.7 | 20.67 | 20.67 | 10.9 2 | 13.5 |
| 3 | 17.67 | 15.18 | | 10.92 | 16.67 | 10.92 | 16.67 | 16.67 | 13.5 | 19.88 |
| 4 | | 10.87 | 13.5 | 10.87 | 13.5 | | | | | |
| 5 | 13.9 | | | 16.83 | 12.9 | | 12.9 | | | |

Figure 11 shows the test results of the compounds of the invention NFKF and NFKL, as well as the dipeptides NF, FK, KF and KL and the tripeptides NFK, FKF and FKL in the pilocarpine model, indicating the time for the occurrence of death after administration control compounds or test compounds.

In the cases of FKF, FKL, NF, FK, KF and KL tests from tables 1-4 above, blank fields are from animals that convulsed and died at the same time.

The results of the tests performed in examples 3 to 8 above show significant and significant in vivo results in dosage ranges of the order of 500 to 1000 μg of compound of the invention per kg of animal. Considering the tests in mice and the conversion to the Human Equivalent Dose mentioned above, effects of the same magnitude are expected in humans in the dosage range of 40 to 80 μg of compound of the invention per kg of human and, Safety ranges, concentrations between 4 to 800 μg/kg for administration to humans are considered in the present invention.

**Example 9. Use of the compound NFKF or NFKL as the synthesis intermediate in the preparation of other compounds**

In the present application, the peptidic compound of the invention is also useful as a synthetic intermediate in obtaining other compounds of pharmaceutical/diagnostic interest. In this and other embodiments the compound is referred to as a "modified peptide", which is unnatural, being artificially modified or obtained by synthesis, including

halides, cyclized, amidated, alkylated, alkoxylated, hydroxylated, PEGylated, other functional groups in any amino acids, or salt forms thereof, as well as modified with an amino acid or peptide, including the non-natural as the d-amino acid forms. In the embodiment of this example, the peptidic compound of the invention has been modified with molecular species useful in diagnostic and/or therapeutic applications, such as Biotin, using techniques known to those skilled in the art. Biotin is a label or tag that can be used in the detection of the ligand target of the invention, in the present case the cannabinoid and/or muscarinic receptors, through the use of anti-biotin antibodies, or avidin/streptavidin detection strategies with enzymes such as horseradish peroxidase, alkaline phosphatase, or fluorescent probes.

The modified compound of the invention in the biotinylated embodiment was used in screening techniques using anti-CB1 receptor antibodies sensitive to conformational changes.

Antibodies sensitive to CB1 receptor activation were generated and characterized by their specificities. For screening compounds, Striatum membranes (5μg per well) were plated on NUNC-Immuno 96-well plates (Nalge-Nunc) and dried at room temperature. Membranes were washed with PBS (phosphate buffered saline) and incubated with or without different ligands (1μM) and the extent of receptor recognition by the antibodies was measured by ELISA. The results of the tests are shown in Table 5, which indicates the% binding to the CB1 receptor in relation to control:

Table 5:

| Assay / Compound | 1 | 2 | 3 | 4 | 5 |
|---|---|---|---|---|---|
| Win | 111.483 | 117.95 | 125.56 | 127.52 | 122.49 |
| Hp | 91.33 | 92.74 | 97.05 | 90.23 | 73.49 |
| NFKF | 90.27 | 84.41 | 90.19 | 77.88 | 65.39 |
| VDHp | 115.72 | 113.62 | 131.029 | 128.99 | 113.05 |
| RVDHp | 103.39 | 111.53 | 110.87 | 114.76 | 105.22 |
| NFKF-Biotin | 89.23 | 85.55 | 83.29 | 84.76 | 97.47 |

Figure 12 shows these results and confirms that the peptidic compound of the invention has also been shown to be an appropriate synthetic intermediate for the preparation of other compounds useful in diagnostic and/or therapeutic applications. The peptide compound of the invention, when modified/protected by biotin, has also been shown to bind to the CB1 receptor in tests with anti-CB1 antibodies sensitive to the activation/conformational changes of the receptor.

In this context, the present co-inventor has already shown (Gupta et al) that μ and δ opioid receptors dimerize with CB1 cannabinoid receptors and that heterodimerization leads to modulation of receptor function. In addition, the use of other CB1 receptor binding substances provides increased recognition of μ opioid receptors, apparently mediated by conformational changes in them. These observations support the idea that conformational changes caused by CB receptor ligands may affect the receptor partner in a heterodimer and that this can be detected using antibodies sensitive to conformational changes. Thus, the test results in this example support the concept of an *in vitro* diagnostic process for protein-protein or peptide-protein interactions that modulate the function of GPCRs (G protein coupled receptors, or G-Protein Coupled Receptors/GPCRs), including heterodimeric interactions.

The results of the present example support one of the invention objects, which is an *in vitro* process for diagnosing the presence, quantity and/or localization of cannabinoid, opioid and/or muscarinic receptors, as well as evaluating the binding of other compounds to these receptors.

Said process comprises contacting the optionally modified compound of the invention with a biological sample containing a cannabinoid, opioid and/or muscarinic receptor. Said method is also useful in the identification and/or quantification of other molecular entities binding to these receptors, and includes a step of detecting said visual, optical, radioactive, chemical, spectroscopic signal connection. Examples include biochemical, cytochemical, histochemical tests. The ligand of the invention is also potentially useful for *in vivo* diagnostic and/or prognostic processes, including contrast and non-contrast imaging tests, for example magnetic resonance spectroscopy (MRS) of molecular entities whose presence and/or amount is modulated by administration of the compound of the invention. This approach is potentially useful in the diagnosis and/or prognosis of degenerative diseases, especially neurodegenerative diseases.

**Example 10. *In vitro* and *in silico* binding/interaction tests of the compound NFKF to the CB1 receptor**

In this example, the *in vitro* affinity profile of one compound of the invention NFKF was evaluated with the cannabinoid receptor CB1.

For this purpose, binding techniques were used to measure the affinity of the NFKF tetrapeptide (powder in 100% purity and prepared as stock solution 10mM in DMSO) by the cannabinoid CB1 receptor at concentrations of 1 and 10 μM. This evaluation was done using the human recombinant CB1 cannabinoid receptor, associated with the radioligand agonist CP 55940 (IC 50 (M) = 1.1 nM; Ki (M) = 0.94 nM; nH = 1). In vitro binding assays were performed using recombinant CHO cells expressing the human CB1 receptor and the [3H] CP 55940 linker (0.5nM concentration; 3.5dM Kd) in incubation for 120 minutes at 37°C using the compound not specific WIN 55212-2 (10mM), with detection by scyntilography counting (Munro et al, 1993).

Results are expressed as the percentage of binding of the specific control according to formula:

$$\frac{\textit{Binding}\text{ specific as measured}}{\textit{Binding}\text{ specific of control}} \times 100$$

And the percentual inhibition of *binding* of specific control obtained in the presence of the compound of the invention is:

$$100 - \left(\frac{\textit{Binding}\text{ specific as measured}}{\textit{Binding}\text{ specific of control}} \times 100\right)$$

IC50 values (concentration that causes half of maximal inhibition of specific control binding) and Hill (nH) coefficients were determined by nonlinear regression of the competition curves generated with the mean values of the replicates using the Hill equation:

$$Y = D + \left(\frac{A - D}{1 + (C/C_{50})^{nH}}\right)$$

Where Y = specific binding, A = asymptotic left of curve, D = asymptotic right of curve, C = compound concentration, $C_{50}=IC_{50}$ and nH = curve coefficient/slope factor. This analysis was performed using the Hill software validated by comparison with data generated by Sigmaplot 4.0 software. Inhibition constants (Ki) were calculated using the equation of Chen Prusoff:

$$K_i = \frac{IC_{50}}{1 + L/K_D}$$

Where L = concentration of the radioligand in the assay; $K_D$ = affinity of the radioligand for the receptor. A graph is used to determine the $K_D$.

Results showing inhibition or stimulation greater than 50% are considered to represent significant effect of the test compound. Results showing inhibition or stimulation between 25% and 50% are indicative of low to moderate effect of the test compound. Results showing inhibition or stimulation of less than 25% can be considered as minor. Results showing inhibition greater than or equal to 50% are indicative non-specific or allosteric effects of the test compound.

The results obtained in duplicate for each concentration tested (1 and 10 μM) are shown in table 6.

Table 6 - In vitro inhibition data of binding of the radioligand agonist CP55940 to the CB1 receptor by NFKF

| *% of inhibition of binding of specific control* | | | |
|---|---|---|---|
| **Concentration** | **1st measurement** | **2nd measurement** | **Mean value** |
| 1 mM | -121.4 | -27.8 | -74.6 |
| 10 mM | -18.9 | -0.9 | -9.9 |

Even if the first measurement were disregarded at 1mM, due to the surprisingly high value, the data of the second measurement at 1mM taken alone would still be considered indicative of effect.

The results indicate that NFKF has greater power to modulate the binding of the respective radioligand when at low concentrations, suggesting that in the range of nM the effects are greater.

These results indicate that the likely mechanism of action associated with the therapeutic profile evidenced for the NFKF tetrapeptide involves allosteric modulation of CB1 cannabinoid receptors, which is consistent with the astonishing / unexpected in vivo test results (examples 3, 4, 5, 6, 7, 8), as well as the results of the *in vitro* experiments described above and the *in silico* experiments described below.

These experimental data indicate that the compound of the invention interacts with and/or modulates the activity of the CB1 receptor and/or its endogenous ligands. Thus the compound of the invention is potentially useful in various metabolic functions, including control of food intake, metabolism of energy and/or lipids, regulation of intestinal motility, immune system, calcium cycle balance, among others. Cannabinoid receptors are widely expressed in the brain, including cortex, hippocampus, amygdala, pituitary, hypothalamus, adrenal gland. CB receptors, particularly CB1, have already been identified in numerous peripheral organs and tissues, including the thyroid gland, reproductive organs, adipose tissue, liver, muscle, and gastrointestinal tract. These results support the use of the compound of the invention in the modulation of metabolic functions and/or neuromodulation.

The cannabinoid receptor 1 (CB1) corresponds to the GPCR (Expressed Receptors to Protein G) most expressed in the human brain and is found at high levels in the Central Nervous System in general (Figure 13). It is activated by endocannabinoids and has been identified as a promising therapeutic target for the treatment of various diseases such as pain and inflammation, multiple sclerosis and neurodegenerative diseases (agonist effect) and obesity, liver fibrosis and nicotine dependence (HUA) et al., 2016).

Figure 13 shows an overview of the three-dimensional structure of a GPCR, in this case, the cannabinoid receptor of subtype 1. The seven transmembrane helices (I-VII), the intracellular loops (ICL1 and ICL2) and the extracellular loops ECL2 and ECL3).

Through the use of Molecular Modeling tools we have evaluated in this example the mode of interaction at the CB1 binding site of one compound of the invention NFKF. In addition, the profile observed was compared with the mode of interaction of other known binders, such as cannabidiol, rimonabant and AM6538.

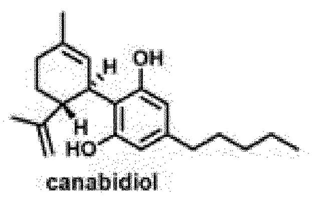
canabidiol

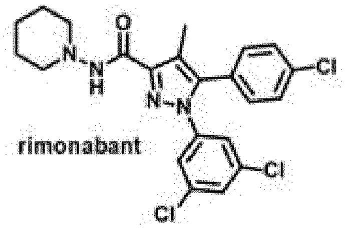
rimonabant

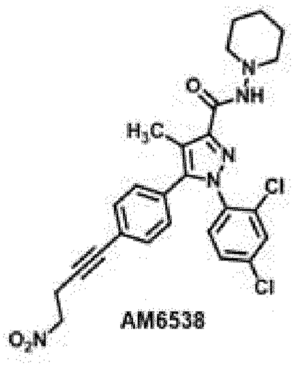
AM6538

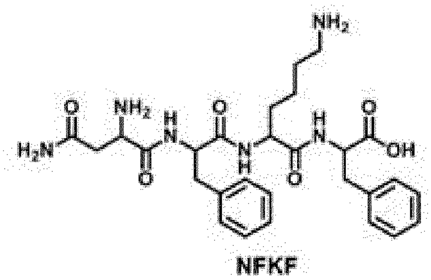
NFKF

The *in silico* tests of the interaction profile of the NFKF tetrapeptide with the CB1 receptor were made from the design and optimization of the ligand structures. Initially, the Percepta program was used to verify the ionization status of

these ligands at plasma pH (pH = 7.4). Subsequently, the construction of ligand structures was performed in the Spartan v. 16 (Wavefunction, Inc.) followed by energy minimization by molecular mechanics.

The selection of the crystallographic structure CB1 for the docking studies was made from the data available in the PDB code 5TGZ, belonging to the species *Homo sapiens* and presenting a resolution of 2.8 Å. Said structure corresponds to the only available CB1 crystal in the database and available at the priority date of this patent application.

The validation of the functions of the GOLD v. 5.1 and conducting docking studies. To identify the most stable complex, three scoring functions were considered in the GOLD Program (ChemScore, Goldscore and ChemPLP). For the realization of molecular anchoring in the GOLD v. 5.5 (CCDC) the location of the active site was required using the co-crystallized binder (AM6538) itself and all amino acids at 6Å distance thereof as reference. Subsequently, molecular anchoring and analysis of the results in the PyMOL v. 1.8.6.2 (Schrödinger, LCC).

Initially, the scoring functions of the GOLD v. 5.5 were evaluated according to the ability to predict protein-binding complexes compatible with experimental results. For this, three scoring functions (Goldscore, Chemscore and ChemPLP) were validated by redocking the reference ligand, AM6538, at the CB1 receptor binding site (PDB 5TGZ). Firstly, docking was performed in the absence of water molecules and, afterwards, trying to estimate how much the water molecules present in the binding site could influence the orientation of the binder, the same evaluation was made in the presence of water molecules. The results obtained by Root Mean Square Deviation RMSD, considering the complexes with higher scores, can be observed in table 7.

For the calculation of the RMSD are considered and compared pairs of atoms of the result of the molecular modeling experiments and the experiments of crystallographic structure. Thus, the square root of the mean between the distances of these atoms is calculated, obtaining the values of the deviation. The smaller the deviation, the greater the approximation between the results, indicating, therefore, the most adequate function to be used (MAIOROV; CRIP-PEN, 1994; HILDEBRANDT et al., 2013).

Table 7: RMSD values obtained from the validation of the scoring functions of the GOLD v. 5.5 for the redocking of AM6538 in the CB1 receptor.

| Function | RMSD | |
|---|---|---|
| | with water | w/o water |
| CHEMPLP | 0.9543 | 0.9134 |
| CHEMSCORE | 1.1762 | 1.1578 |
| **GOLDSCORE** | **0.9384** | **0.8864** |

As indicated in Table 7, the function which showed the lowest RMSD values, and which proved to be most suitable for the docking experiments, was Goldscore in the absence of water molecules at the binding site, indicating that such molecules do not influence the mode of the ligand.

Figure 14 shows visually the overlap between the crystallographic structure complex (PDB 5TGZ) and the resulting complex from the AM6538 redocking experiment using the validated methodology.

The complex resulting from the redocking of AM6538 in CB1 obtained a score of 87.39 and the interaction profile observed was consistent with that described in the literature (HUA et al., 2016), with several hydrophobic interactions with amino acid residues such as: Phe-102, Phe-170, Phe-174 and Phe-379, as well as leucine, methionine, cysteine and valine residues (see Figure 15).

On the other hand, for the rimonabant and cannabidiol, the interaction profiles observed were very similar to the AM6538, respectively, presenting scores of 73.43 and 54.91 (Figures 16 and 17, respectively).

In the docking experiment of one embodiment of the compound of the invention (NFKF tetrapeptide) its structure was analyzed for its ionizing state at plasma pH (pH = 7.4) in the Percepta program, which indicated the structure shown below, which was used for docking studies

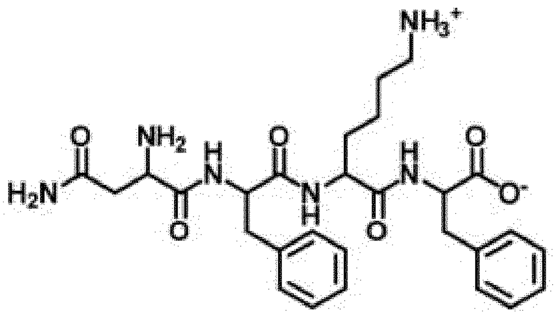

The resulting docking complex of the NFKF tetrapeptide in CB1 scored 100.66, substantially higher than the score achieved by the reference ligands. This fact can be explained by additional hydrogen bonds observed between the tetrapeptide and the residues Ser-123, Thr-197, Ser-167 and Ser-383. In addition, the hydrophobic interactions observed for AM6538 are also present in the NFKF interaction mode (Figure 18).

Together, the results of these experiments show that the observed interaction profile for NFKF, with similar interactions to the reference ligands, as well as additional interactions that resulted in a higher score obtained in the docking studies, suggest that the NFKF tetrapeptide is a potential ligand of cannabinoid receptors of subtype 1.

**Example 11. *In vitro* and *in silico* binding/interaction tests of compound NFKF to CB2 receptor**

In this example, the in vitro affinity profile of one compound of the invention, NFKF tetrapeptide, was evaluated with the cannabinoid receptor CB2.

For this purpose, binding techniques were used to measure the affinity of the NFKF tetrapeptide (powder in 100% purity and prepared as stock solution 10mM in DMSO) by the cannabinoid receptor CB2 at concentrations of 1 and 10 μM. This evaluation was done using the recombinant human CB2 cannabinoid receptor associated with the radioligand agonist WIN 55212-2 (IC 50 (M) = 1.5 nM; Ki (M) = 0.96 nM; nH=0.9). In vitro binding assays were performed using recombinant CHO cells expressing human CB2 receptor and [3H] WIN55212-2 linker (0.8nM concentration; 1.5nM Kd) in incubation for 120 minutes at 37°C using non-specific compound WIN 55212-2 (5mM), with detection by scyntilography counting (Rinaldi-Carmona et al, 1996).

The results are expressed as indicated in example 10 above.

The results obtained in duplicate for each concentration tested (1 and 10 μM) are shown in table 8.

Table 8 - *In vitro* inhibition data of binding of radioligand agonist WIN 55212-2 to CB2 receptor by NFKF

| *% of inhibition of binding of specific control* | | | |
|---|---|---|---|
| **Concentration** | **1st measurement** | **2nd measurement** | **Mean value** |
| 1 mM | 3.5 | 0.3 | 1.9 |
| 10 mM | 9.5 | 13.1 | 11.3 |

The results indicate that NFKF did not provide significant modulation in binding of the respective radioligand under the conditions tested.

Taking these results together with the results of the experiments described in example 10, one arrived at interesting conclusions. Although the NFKF peptide has demonstrated a selective ligand of CB2 cannabinoid receptors, as indicated by the results of the docking experiments for this receptor, in vitro test results indicate that this tetrapeptide is not able to bind to this cannabinoid receptor in a significant manner. However, an interaction of the compound of the invention with the allosteric type CB2 receptor cannot be ruled out. Thus, the test results indicated a positive modulation of CB2 receptor, although moderate at the concentrations tested, suggest a combined (allosteric) modulation effect which is negative for CB1 and positive for CB2.

In addition, in vitro test results (examples 9, 10 and / or 11) demonstrate that the compound of the invention can be used as a binder of diagnostic interest, such as, for example, radioactive or chromophore labeling for subsequent identification of binding sites in cells and/or tissues.

In order to enable *in silico* experiments of the interaction of one compound of the invention NFKF tetrapeptide, with the CB2 receptor, a three-dimensional model of the CB2 receptor was first constructed, for subsequent docking experiments.

In this embodiment, the construction of the 3D model of CB2 was done from a search in the UniProt database of the amino acid sequence of this receptor. The criterion used for sequence selection was the species (Homo sapiens). Thus, the sequence selected for carrying out the molecular modeling studies was that of code P34972.

Next, the Template Identification tool of the SwissModel server was used to identify and select the protein template. The sequence identified by the server as having the highest structural identity to the human CB2 sequence was that of PDT 5TGZ code (HUA et al., 2016) belonging to the species Homo sapiens, corresponding to the CB1 receptor.

The target and template sequences were then aligned using the ClustalW2 software linked to the UniProt database to compare the sequences to establish the percentage of identity between them. The 3D homology model of human CB2 was constructed using the Automated Mode tool available on the Swiss-model server page and, for validation of the generated model, the overall structural quality and stereochemical quality of the model were analyzed through the value presented for the GMQE parameter and the Ramachandran graph analysis.

After the creation of the 3D model for CB2, docking studies were performed for the NFKF tetrapeptide as well as for cannabidiol, rimonabant and AM6538.

After identification and selection of the human CB2 receptor sequence in UniProt and the identification of the template protein (PDB code 5TGZ) from the Template Identification tool on the Swiss-Model server, alignment was made between the two sequences in the program ClustalW2. The identity between the two sequences was 46.18%, which makes possible the use of comparative modeling as a tool to obtain the 3D structure of the human CB2 receptor, as recommended in previously published studies (MORGAN and HURLEY, 2015), which affirm be necessary the identity of at least 30% between primary sequences for the creation of homology models.

The stereochemical quality of the model was analyzed using the Ramachandran graph (Figure 19B). The Ramachandran graph corresponds to a mathematical model, which relates the dihedral angles $\Phi$ (angle C-N-C$\alpha$-C) on the x-axis and $\Psi$ (angle N-C$\alpha$-C-N) on the y-axis. This graph is divided into regions capable of representing the probability of a combination between the angles $\Phi$ and $\Psi$ (RAMACHANDRAN; RAMAKRISHNAN; SASISEKHARAN, 1963). These regions are classified as: favorable, permitted, generously permitted and prohibited.

The Ramachandran graph constructed for the human CB2 model showed the presence of approximately 96% of the amino acid residues in the most favorable regions and more than 4% of the residues in acceptable regions. No amino acid residue was found in forbidden regions, indicating the stereochemical validation of the model created. After the validation of the model, docking studies on CB2 of the NFKF tetrapeptide, the AM6538 antagonist, the cannabidiol endogenous ligand and the rimonabant antagonist were performed.

For all compounds analyzed, the scores obtained in the studies were lower than those achieved for CB1, from 61.53 for AM6538, 72.33 for NFKF tetrapeptide, 43.31 for cannabidiol, and finally 57.40 for rimonabant.

Since, in a simplistic way, punctuation values can be considered as a sum of the interactions identified between the protein and the linker, the lower scores can then be explained by a lower number of interactions at the binding site, although the major hydrophobic interactions observed in CB1 are maintained in CB2 as can be seen in Figures 20-23.

It is also important to note that for the NFKF tetrapeptide the hydrogen bonding interactions indicated in CB1 do not occur in CB2, contributing to its lower CB2 score (Figure 23), although this peptide presented another hydrogen bond with the Lys-192 residue.

Table 9 briefly summarizes the comparison between the scores obtained by the docking studies in both receivers (CB1 and CB2) in Example 10 and in this example.

Table 9: Comparison between the scores obtained in the docking studies for CB1 and CB2 receptors.

| | **Scores** | |
|---|---|---|
| **Ligands** | **CB1** | **CB2** |
| Cannabidiol | 54.91 | 43.31 |
| Rimonabant | 73.43 | 57.40 |
| AM6538 | 87.39 | 61.53 |
| NFKF | 100.66 | 72.33 |

The results of the experimental tests described in examples 9, 10 and/or 11 above allowed the replication, for the other compounds tested, of interaction mode of antagonist AM6538 at the CB1 receptor binding site, as described in the literature by Hua et al. In addition, the results provided validation of the docking methodology for experiments with NFKF tetrapeptide compared to cannabidiol and rimonabant antagonist, and it is therefore possible to predict their interaction profiles in CB1.

Construction and validation of a 3D model by homology of the CB2 receptor also resulted in visualization of the behaviour of all of the ligands mentioned at the binding site of such receptor, providing a comparative analysis with results

obtained with CB1.

In summary, the results of the *in vitro* and *in silico* experiments of examples 9, 10 and 11 indicate that the compounds of the invention are CB1 receptor ligands. In addition, less favourable interactions of the NFKF compound were evidenced in CB2 receptors, indicating a possible selectivity profile. The results of the experiments of Examples 10 and 11, notably those shown in Figures 17 and 23, show that NFK amino acids participate much more strongly in the binding of CB1 and CB2 than the amino acid F in the C-terminal position, indicating that this tripeptide is a potential candidate for ligand/modulator of such receptors. The results of the in vivo tests shown in figure 7 are in line with this statement.

## Example 12. Analgesic or neuropathic pain modulating pharmaceutical composition comprising the compound of the invention - results of *in vivo* tests

The compounds of the present invention have also been tested as analgesics or neuropathic pain modulators. To that end, the compounds PVNFKF, PVNFK, VNFKF, NFKF were tested in the pain threshold model. The pain threshold was measured using a pressure apparatus (Ugo Basile, Italy), essentially as described (Randall & Selitto, 1957). Briefly, a force of increasing magnitude (16 g/s) was applied to the back of the right paw of rats. When animals react by kicking, the force in grams required to induce this response is the threshold of pain. Antinociceptive activity was expressed as the increase in pain threshold compared to control animals.

This induced pain model does not require concomitant administration of other substances. The compounds of the invention were administered orally to the animals at doses of 0.5 to 0.25 mg/kg and saline was used as a control.

Table 10 shows the strength results in grams for pain threshold:

Table 10:

| Immediate | | | | | | | |
|---|---|---|---|---|---|---|---|
| 1 | Control | 70 | 70 | 60 | 65 | 70 | 70 |
| 2 | PVNFKF | 75 | 65 | 60 | 70 | 80 | 75 |
| 3 | PVNFK | 70 | 75 | 75 | 65 | 60 | 75 |
| 4 | VNFKF | 70 | 65 | 65 | 70 | 75 | 75 |
| 5 | NFKF | 70 | 75 | 75 | 75 | 80 | 60 |

| After 3h | | | | | | | |
|---|---|---|---|---|---|---|---|
| 1 | Control | 30 | 55 | 55 | 50 | 45 | 40 |
| 2 | PVNFKF | 45 | 30 | 55 | 45 | 65 | 55 |
| 3 | PVNFK | 55 | 45 | 40 | 55 | 60 | 40 |
| 4 | VNFKF | 85 | 40 | 35 | 50 | 50 | 50 |
| 5 | NFKF | 80 | 70 | 80 | 75 | 70 | 70 |

Figure 24 shows the pain threshold results, force in grams applied to the back of the right paw of the animals. Significant and very evident is the analgesic effect provided by compound NFKF, which increased the pain threshold after 3h of administration when compared to the immediate pain threshold. In addition, compound NFKF was shown to be significantly and substantially superior to PVNFKF, with a 50.1% greater pain threshold in animals administered NFKF compared to PVNFKF.

In this context, the present co-inventor has already demonstrated (Gupta et al) that $\mu$ and $\delta$ opioid receptors dimerize with CB1 cannabinoid receptors and that heterodimerization leads to modulation of receptor function. In addition, the use of other CB1 receptor binding substances provides increased recognition of $\mu$ opioid receptors, apparently mediated by conformational changes in them. These observations support the idea that conformational changes caused by CB receptor ligands can affect the receptor partner in a heterodimer, providing therapeutic effects.

The evident analgesic effect data of the compound of the invention in the present example suggest that the compound of the invention provides similar effect to that of Hp when combined with morphine, i.e. with a lower dose of morphine in combination with Hp, the same analgesic effects. The use of the compound of the invention in combination with morphine, therefore, may contribute to lessening important implications on the addiction and tolerance that morphine causes.

## Example 13. Pharmaceutical composition for the treatment of multiple sclerosis comprising the compound NFKF - results of *in vivo* tests

Although the exact etiology of Multiple Sclerosis is still unclear, it is speculated that auto-reactive T lymphocytes play a central role in its pathophysiology. The most common animal model of Multiple Sclerosis is experimental autoimmune encephalomyelitis (EAE), because it shares many physiological and clinical aspects. There are several models of EAE that reflect different clinical, immunological and histological aspects of human multiple sclerosis. The active induction model of EAE in mice is robust and provides replicable results and is particularly useful in the search for therapeutic agents through the use of transgenic mice challenged by autoimmune neuroinflammation. The EAE model is often used as evidence of the efficacy of novel therapeutic strategies for Multiple Sclerosis.

In this embodiment, transgenic mice knockout of the Endopeptidase 24.15 gene were challenged with MOG (Myelin Oligodendrocyte Glycoprotein), with administration of the compound of the invention NFKF or saline as a control. After the induction of the disease, a daily evaluation of the symptoms was made, according to table 11 below.

Table 11. Clinical Score System

| Score | Clinical Signal | Comment |
|---|---|---|
| 0 | None | Normal walk, tail moves and can be lifted, the tail wraps around a rounded object if the animal is hung by the base of the tail |
| 1 | Partially limp tail | Normal walk, fallen tail tip |
| 2 | Tail paralyzed | Normal walk, fallen tail |
| 3 | Back limb with paresis, uncoordinated movement | Uncoordinated walk, limp tail, hind limbs respond to pinching |
| 4 | A paralyzed hind limb | Non-coordinated walk with a trailing hind limb, limp tail, a hind limb does not respond to pinching |
| 5 | Both paralyzed hindquarters | Non-coordinated walk with both hind limbs dragging, limp tail, both hind limbs do not respond to pinching |
| 6 | Both back limbs paralyzed, weakness in the limbs | Non-coordinated walk with the limbs striving to pull the body, there is reflex in the front limbs after pinching, limp tail |
| 7 | Both hind limbs paralyzed, one front limb paralyzed | Animal cannot move, there is reflex in the fore limb after pinching, limp tail |
| 8 | Both hind and front limbs paralyzed | Animal cannot move, front limbs do not respond to pinching, limp tail |
| 9 | Dying | No movement, breathing altered |
| 10 | Death | |

Figure 25 shows the results of experiments conducted according to the model described above, by administration of saline (control) or NFKF to knock out mice. In A, the Clinical Score curve of the Wild Type (WT) mice, that is, normal, submitted to EAE induction with MOG; In B, the Clinical Score curve of the mice knock out of the gene endopeptidase 24.15, that is, transgenic and prone to the symptoms of Multiple Sclerosis, is shown in the EAE model submitted to MOG induction; In C, the Clinical Score curve of the mice knock out 24.15, that is, transgenic and prone to the symptoms of Multiple Sclerosis in the EAE model, were submitted to induction with MOG and NFKF neuroprotection. * $P < 0.05$ 24.15 KO + NFKF vs WT; + $P < 0.05$ 24.15 KO vs WT. The results show an impressive reduction of clinical symptoms in animals treated with the compound of the invention, which, although being transgenic and deficient in the endopeptidase gene 24.15, exhibited behaviour very close to that of normal animals. These results constitute "proof of principle" of the efficacy of this new therapeutic strategy for the curative or prophylactic treatment of Multiple Sclerosis.

It should also be noted that normal animals submitted to MOG induction have increased levels of interferon and interleukin 17; and in the knockout animals these markers - both proinflammatory - are well increased. These data, together with the response timing profile of NFKF-treated transgenic animals (Figure 25), are consistent with effects on the immune response and/or anti-inflammatory effect or the compound of the invention. In this context, it is known that the cannabinoid system is involved in a variety of immune cells whose activation through receptors triggers a series of signal transductions that affect the transcription and production of cytokines and chemokines. It is known that the modulation of cannabinoid receptors inhibits migration activities of various types of immune cells. Furthermore, the influence of the

cannabinoid system on immune function can occur in sites located in the periphery and also within the central nervous system, which is why the literature suggests that the cannabinoid system plays an important role in the fine regulation of homeostatic immune balance. The compound of the invention, having been shown to modulate the cannabinoid system, may interfere with this system, being potentially useful in a variety of inflammatory, immune, autoimmune conditions.

## Claims

1. Compound **characterized by** formula: NFKF, NFKL, its salts, and/or combinations thereof.

2. Compound according to claim 1 for use as a medicament for a mammal.

3. Compound according to claim 1 for use in the treatment of disorders of energy and/or lipid metabolism; hypertension, intestinal motility disorders; the immune system; the calcium cycle equillibrium; disorders of the thyroid gland, disorders of reproductive organs, obesity, diabetes, diseases or disorders of the immune system/inflammation, osteopeny, osteoporosis, cancer.

4. Compound according to claim 1 for use as an analgetic medicament for a mammal and/or for use in the treatment of migraine, pain, neuropathic pain in a mammal.

5. Compound according to claim 1 for use in the curative or prophylactic treatment of convulsions in a mammal.

6. Compound according to claim 1 for use as a neuromodulative or neuroprotective medicament for a mammal, and/or for the treatment of psychiatric diseases, anxiety, squizophreny or bipolar disorder, Alzheimer, Parkinson, autism, epilepsy, multiple sclerosis.

7. Pharmaceutical composition **characterized by** comprising a pharmaceutically acceptable vehicle; and, as active ingredient, the compound of claim 1.

8. Pharmaceutical composition according to claim 7 for use as medicament for a mammal.

9. Pharmaceutical composition according to claim 7 for use in a method of modulating the metabolic function in a mammal.

10. Pharmaceutical composition according to claim 7 for use for curative or prophylactic treatment of convulsions in a mammal.

11. Pharmaceutical composition according to claim 7 for use for curative or prophylactic treatment for neuromodulation and/or neuroprotection in a mammal.

12. Pharmaceutical composition according to claim 7 for use for curative or prophylactic treatment of multiple sclerosis in a mammal.

13. Pharmaceutical composition according to claim 7 for use according to any one of claims 8-12 **characterized in that** the composition is in the form of a tablet, gel, oral liquid or syrup, capsule, suppository, injectable solution, inhalable or adhesive form.

## Patentansprüche

1. Chemische Verbindung, **gekennzeichnet durch** die Formel: NFKF, NFKL, deren Salze und/oder Kombinationen davon.

2. Chemische Verbindung nach Anspruch 1 zur Verwendung als Arzneimittel für ein Säugetier.

3. Chemische Verbindung nach Anspruch 1 zur Verwendung bei der Behandlung von Störungen des Energie- und/oder Lipidstoffwechsels, Bluthochdruck, Darmmotilitätsstörungen, des Immunsystems, des Kalziumhaushalts, Störungen der Schilddrüse, Störungen der Fortpflanzungsorgane, Fettleibigkeit, Diabetes, Erkrankungen oder Störungen des

Immunsystems/Entzündungen, Osteopenie, Osteoporose, Krebs.

4. Chemische Verbindung nach Anspruch 1 zur Verwendung als Analgetikum für ein Säugetier und/oder zur Verwendung bei der Behandlung von Migräne, Schmerzen, neuropathischen Schmerzen bei einem Säugetier.

5. Chemische Verbindung nach Anspruch 1 zur Verwendung bei der kurativen oder prophylaktischen Behandlung von Konvulsionen bei einem Säugetier.

6. Chemische Verbindung nach Anspruch 1 zur Verwendung als neuromodulierendes oder neuroprotektives Arzneimittel für ein Säugetier und/oder zur Behandlung von psychiatrischen Erkrankungen, Angstzuständen, Schizophrenie oder bipolaren Störungen, Alzheimer, Parkinson, Autismus, Epilepsie, Multipler Sklerose.

7. Pharmazeutische Zusammensetzung, **dadurch gekennzeichnet, dass** sie einen pharmazeutisch verträglichen Träger und als Wirkstoff die Verbindung nach

8. Pharmazeutische Zusammensetzung nach Anspruch 7 zur Verwendung als Arzneimittel für ein Säugetier.

9. Pharmazeutische Zusammensetzung nach Anspruch 7 zur Verwendung in einem Verfahren zur Modulation der Stoffwechselfunktion bei einem Säugetier.

10. Pharmazeutische Zusammensetzung nach Anspruch 7 zur Verwendung zur kurativen oder prophylaktischen Behandlung von Konvulsionen bei einem Säugetier.

11. Pharmazeutische Zusammensetzung nach Anspruch 7 zur Verwendung zur kurativen oder prophylaktischen Behandlung zur Neuromodulation und/oder Neuroprotektion bei einem Säugetier.

12. Pharmazeutische Zusammensetzung nach Anspruch 7 zur Verwendung zur kurativen oder prophylaktischen Behandlung von Multipler Sklerose bei einem Säugetier.

13. Pharmazeutische Zusammensetzung nach Anspruch 7 zur Verwendung nach einem der Ansprüche 8 bis 12, **dadurch gekennzeichnet, dass** die Zusammensetzung in Form einer Tablette, eines Gels, einer oralen Flüssigkeit oder eines Sirups, einer Kapsel, eines Zäpfchens, einer injizierbaren Lösung, einer inhalierbaren oder einer adhäsiven Form vorliegt.

## Revendications

1. Composé **caractérisé par** la formule : NFKF, NFKL, ses sels et/ou des combinaisons de ceux-ci.

2. Composé selon la revendication 1 pour utilisation en tant que médicament pour un mammifère.

3. Composé selon la revendication 1 pour utilisation dans le traitement de troubles de l'énergie et/ou du métabolisme lipidique ; l'hypertension, les troubles de la motilité intestinale ; le système immunitaire ; l'équilibre du cycle du calcium ; les troubles de la glande thyroïde, les troubles des organes reproducteurs, l'obésité, le diabète, les maladies ou troubles du système immunitaire/inflammation, l'ostéopénie, l'ostéoporose, le cancer.

4. Composé selon la revendication 1 pour utilisation en tant que médicament analgésique pour un mammifère et/ou pour utilisation dans le traitement de la migraine, de la douleur, de la douleur neuropathique chez un mammifère.

5. Composé selon la revendication 1 pour utilisation dans le traitement curatif ou prophylactique des convulsions chez un mammifère.

6. Composé selon la revendication 1 pour utilisation en tant que médicament neuromodulateur ou neuroprotecteur pour un mammifère, et/ou pour le traitement de maladies psychiatriques, l'anxiété, la schizophrénie ou le trouble bipolaire, Alzheimer, Parkinson, l'autisme, l'épilepsie, la sclérose en plaques.

7. Composition pharmaceutique **caractérisée en ce qu'**elle comprend un vecteur pharmaceutiquement acceptable ; et, en tant que principe actif, le composé selon la revendication 1.

**8.** Composition pharmaceutique selon la revendication 7 pour utilisation en tant que médicament pour un mammifère.

**9.** Composition pharmaceutique selon la revendication 7 pour utilisation dans un procédé de modulation de la fonction métabolique chez un mammifère.

**10.** Composition pharmaceutique selon la revendication 7 pour utilisation pour le traitement curatif ou prophylactique des convulsions chez un mammifère.

**11.** Composition pharmaceutique selon la revendication 7 pour utilisation pour le traitement curatif ou prophylactique pour neuromodulation et/ou neuroprotection chez un mammifère.

**12.** Composition pharmaceutique selon la revendication 7 pour utilisation pour le traitement curatif ou prophylactique de la sclérose en plaques chez un mammifère.

**13.** Composition pharmaceutique selon la revendication 7 pour utilisation selon l'une quelconque des revendications 8-12 **caractérisée en ce que** la composition se présente sous la forme d'un comprimé, d'un gel, d'un liquide oral ou d'un sirop, d'une capsule, d'un suppositoire, d'une solution injectable, d'une forme inhalable ou adhésive.

Fig. 1

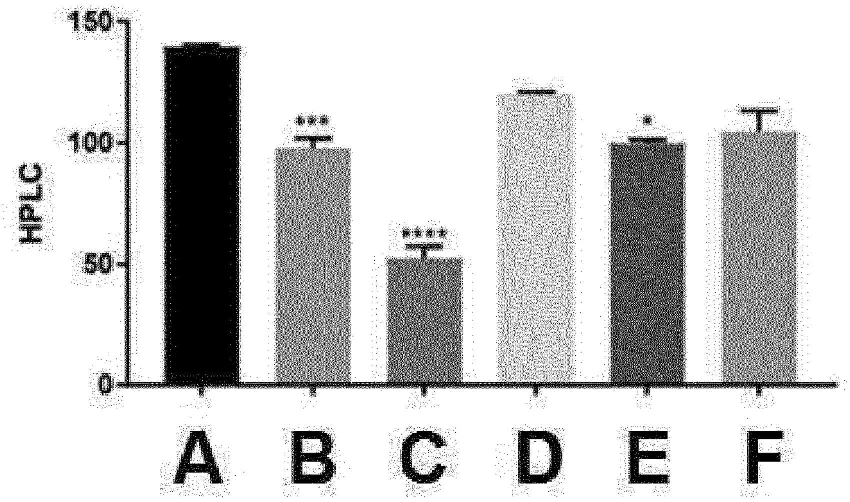


Fig. 2

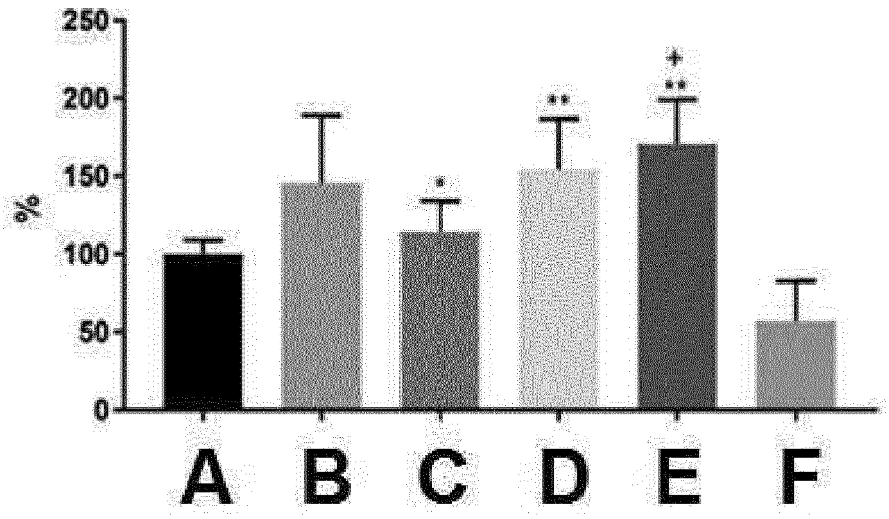

Fig. 3

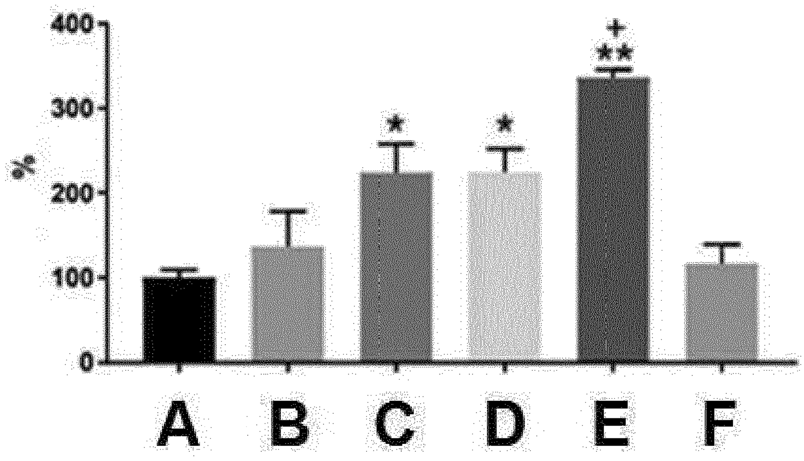
400
300
200
100
0
%
+
**
*
*
A
B
C
D
E
F

Fig. 4

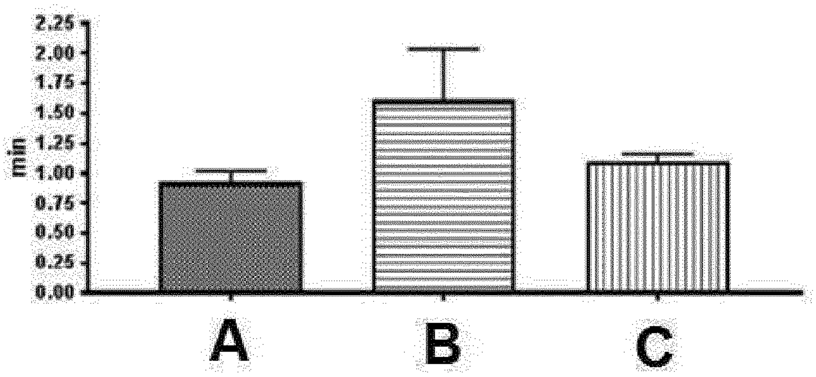
2.25
2.00
1.75
1.50
1.25
1.00
0.75
0.50
0.25
0.00
min
A
B
C

Fig. 5

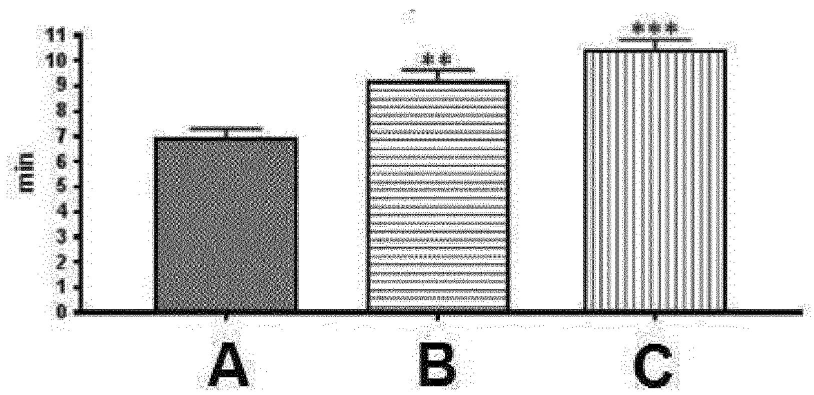


Fig. 6

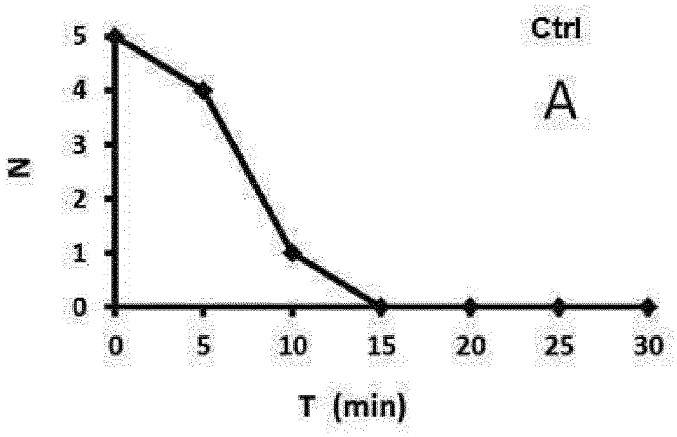

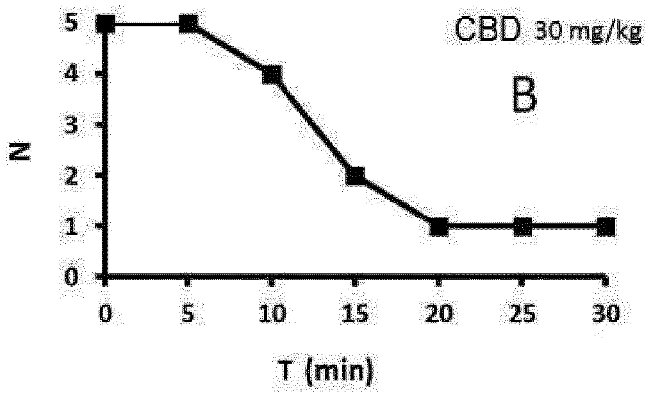
CBD 30 mg/kg
B
N
5
4
3
2
1
0
0
5
10
15
20
25
30
T (min)

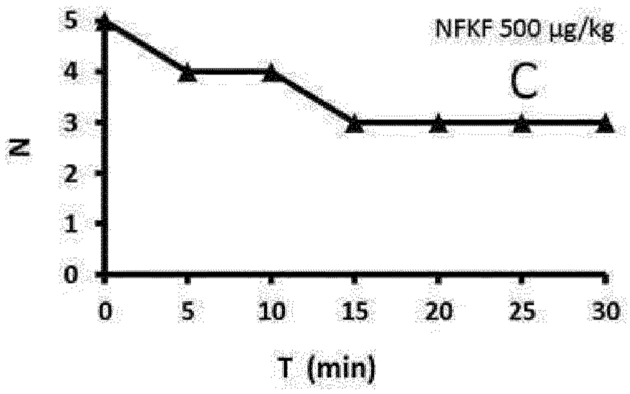
NFKF 500 µg/kg
C
N
5
4
3
2
1
0
0
5
10
15
20
25
30
T (min)

Fig. 6 (cont.)

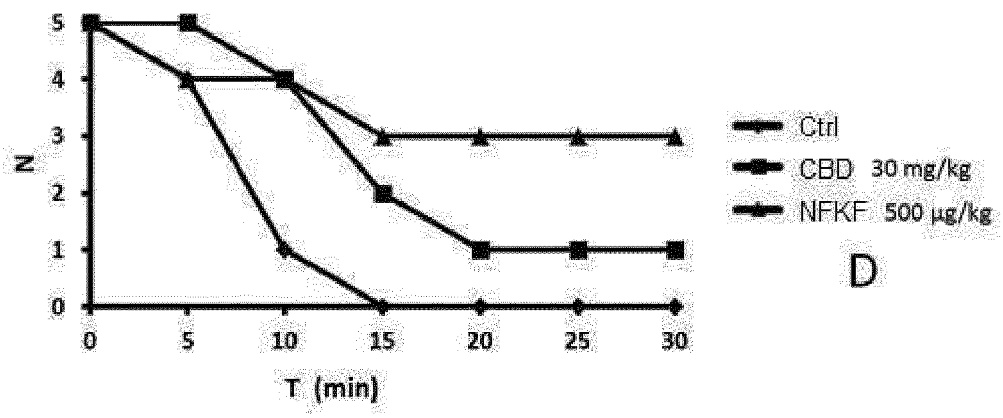
N
5
4
3
2
1
0
0
5
10
15
20
25
30
T (min)
Ctrl
CBD 30 mg/kg
NFKF 500 µg/kg
D

Fig. 7

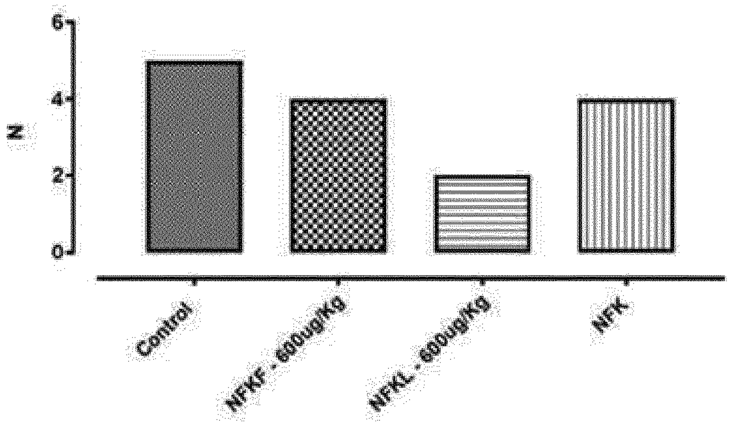
6
4
2
0
N
Control
NFKF - 600ug/Kg
NFKL - 600ug/Kg
NFK

Fig. 8

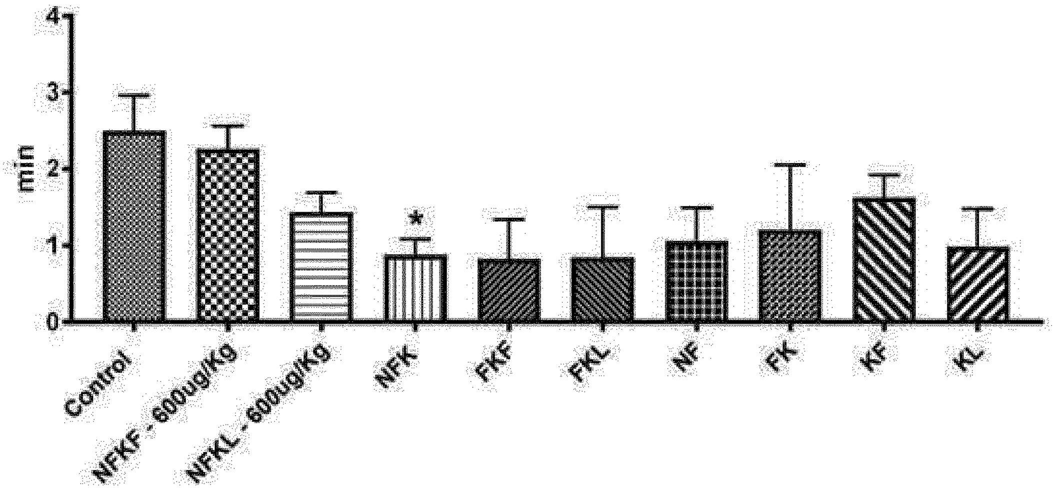
4
3
2
1
0
min
*
Control
NFKF - 600ug/Kg
NFKL - 600ug/Kg
NFK
FKF
FKL
NF
FK
KF
KL

Fig. 9

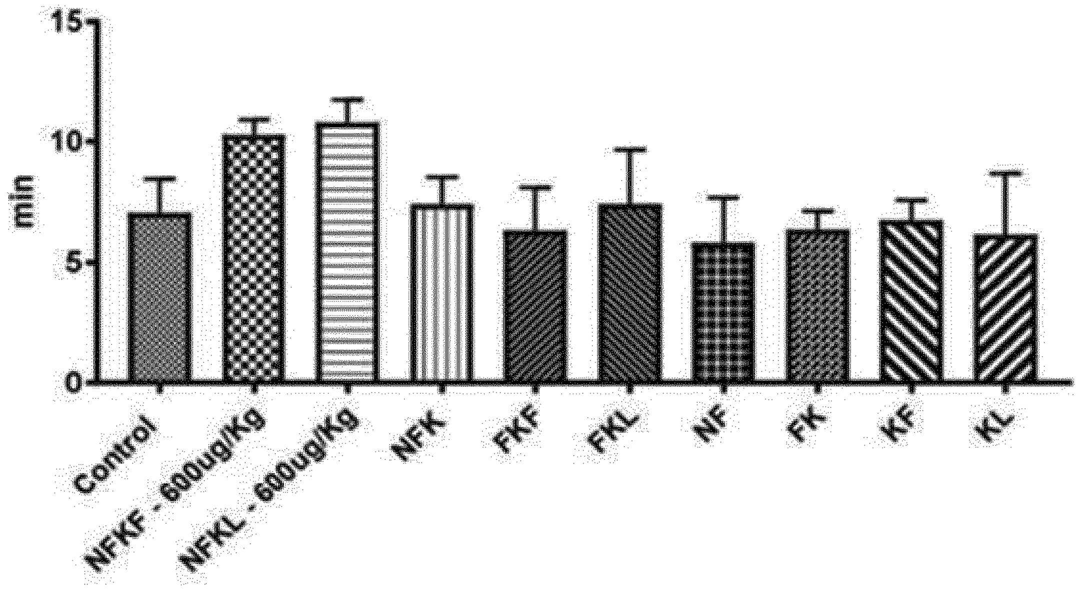
15
10
5
0
min
Control
NFKF - 600ug/Kg
NFKL - 600ug/Kg
NFK
FKF
FKL
NF
FK
KF
KL

Fig. 10

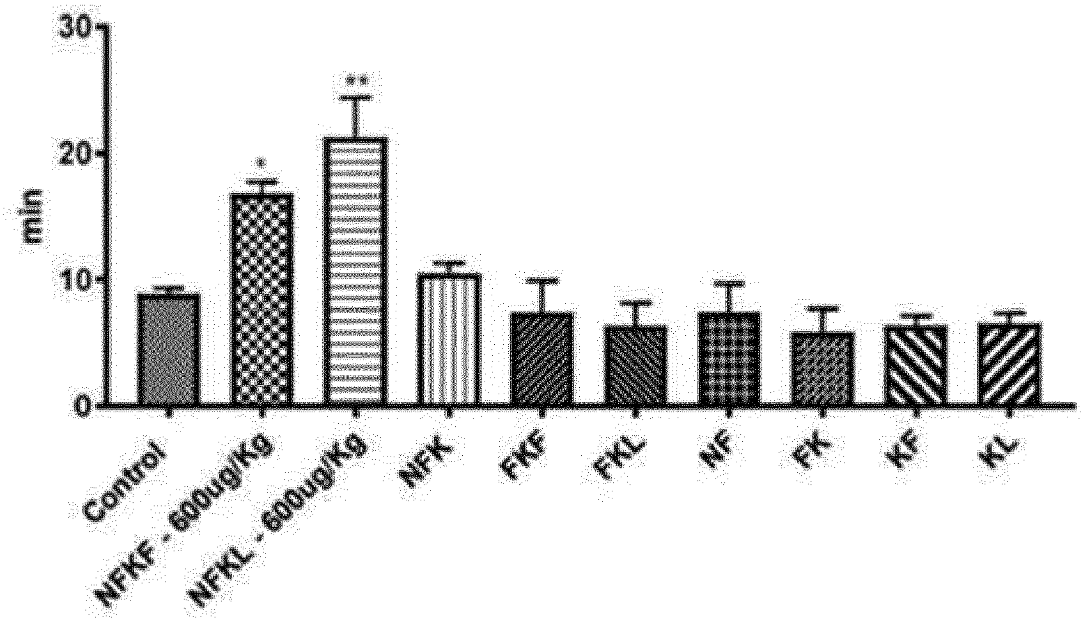
30
20
10
0
min
*
**
Control
NFKF - 600ug/Kg
NFKL - 600ug/Kg
NFK
FKF
FKL
NF
FK
KF
KL

Fig. 11

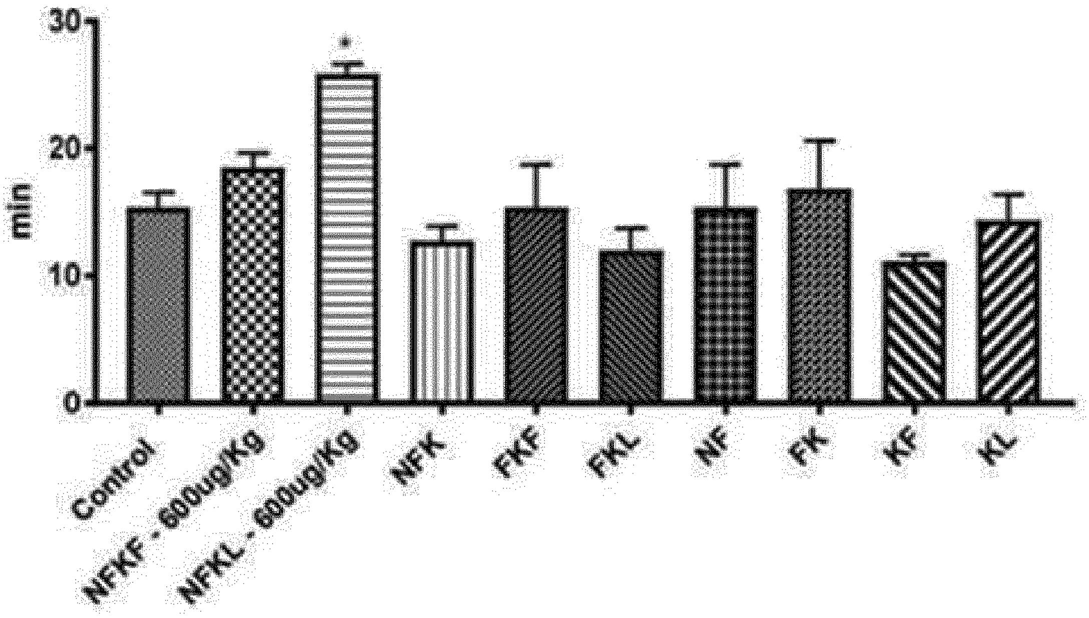
30
20
10
0
min
*
Control
NFKF - 600ug/Kg
NFKL - 600ug/Kg
NFK
FKF
FKL
NF
FK
KF
KL

Fig. 12

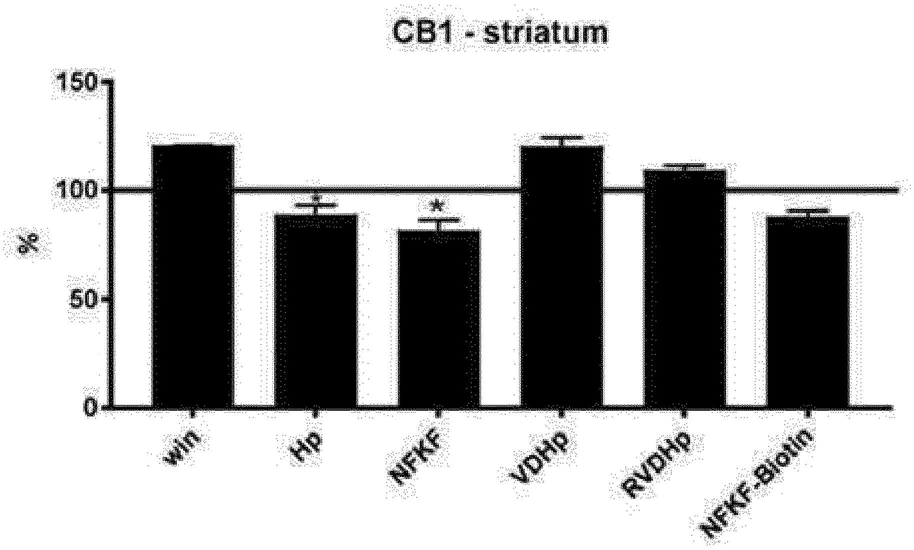
CB1 - striatum
150
100
50
0
%
*
*
win
Hp
NFKF
VDHp
RVDHp
NFKF-Biotin

Fig. 13

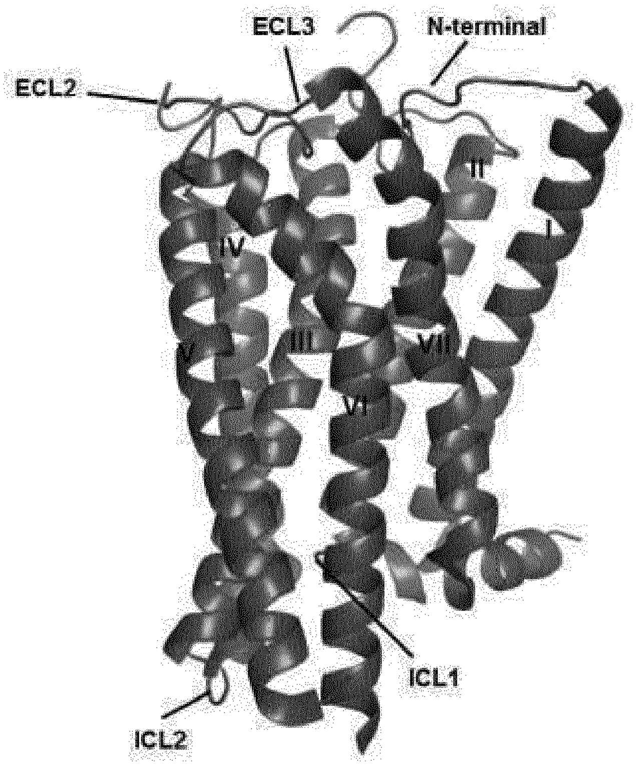


Fig. 14

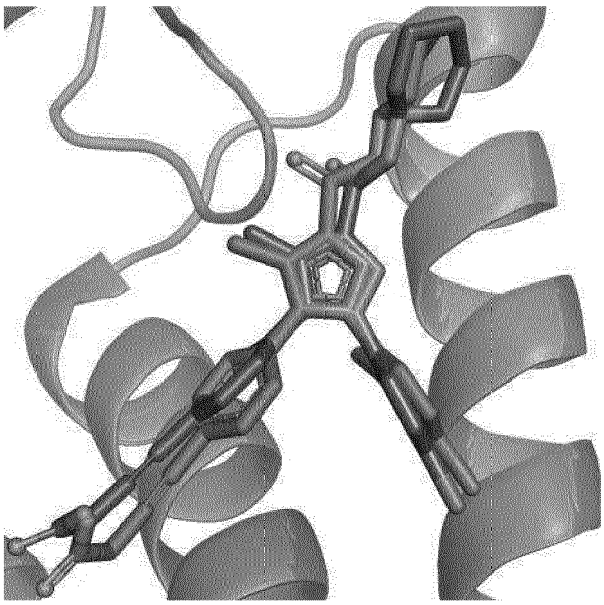

Fig. 15

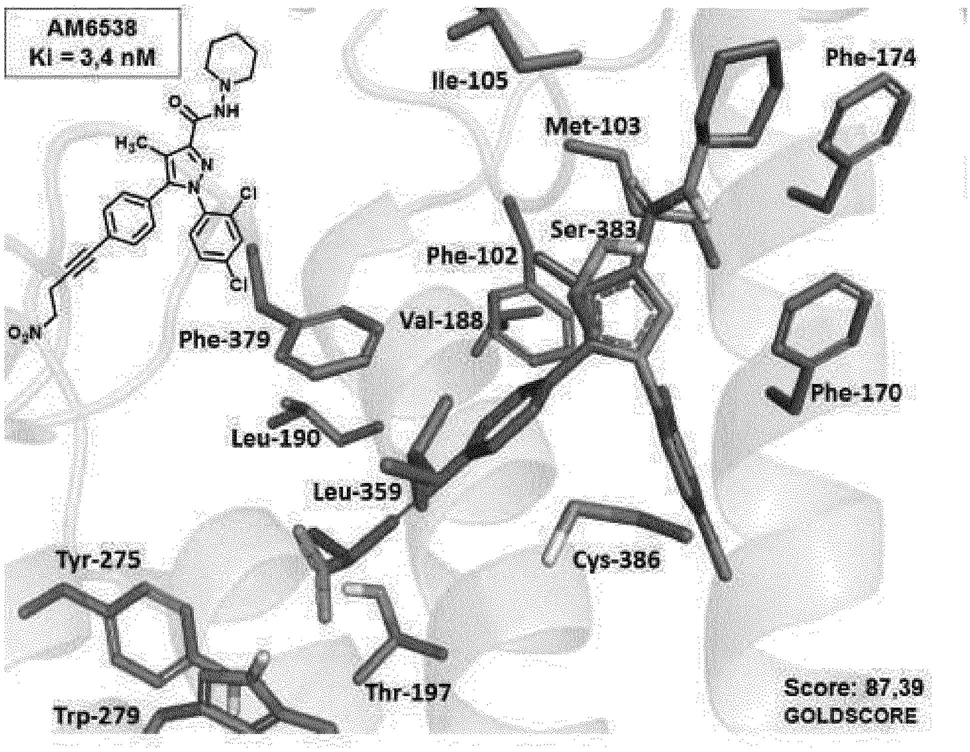
AM6538
Ki = 3,4 nM
Ile-105
Phe-174
Met-103
Ser-383
Phe-102
Val-188
Phe-379
Phe-170
Leu-190
Leu-359
Cys-386
Tyr-275
Thr-197
Trp-279
Score: 87,39
GOLDSCORE

Fig. 16

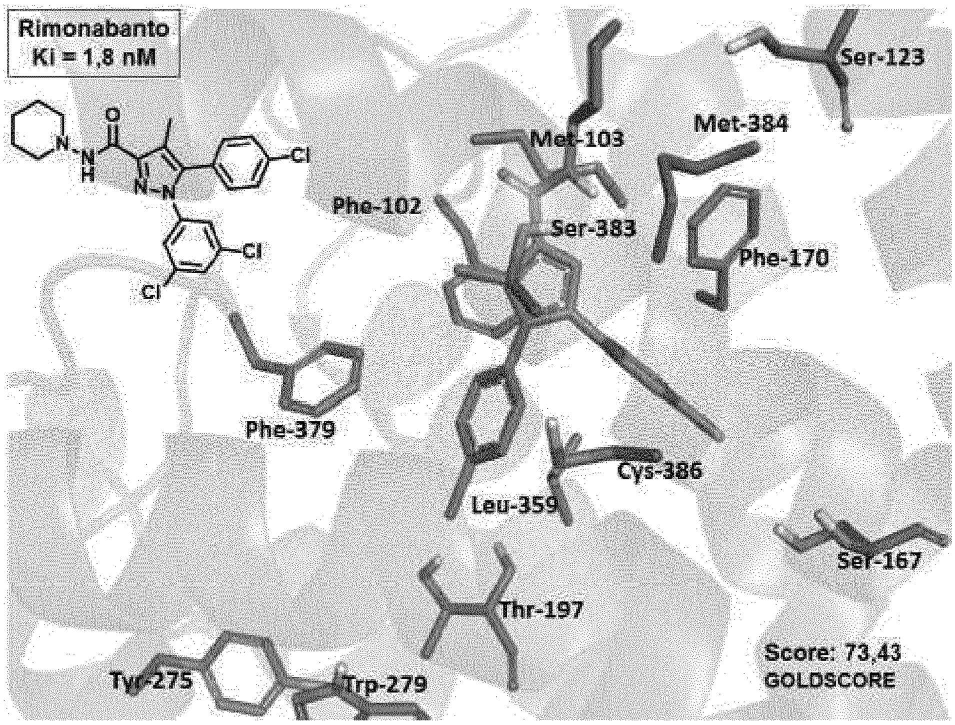
Rimonabanto
Ki = 1,8 nM
Ser-123
Met-103
Met-384
Phe-102
Ser-383
Phe-170
Phe-379
Cys-386
Leu-359
Ser-167
Thr-197
Tyr-275
Trp-279
Score: 73,43
GOLDSCORE

Fig. 17

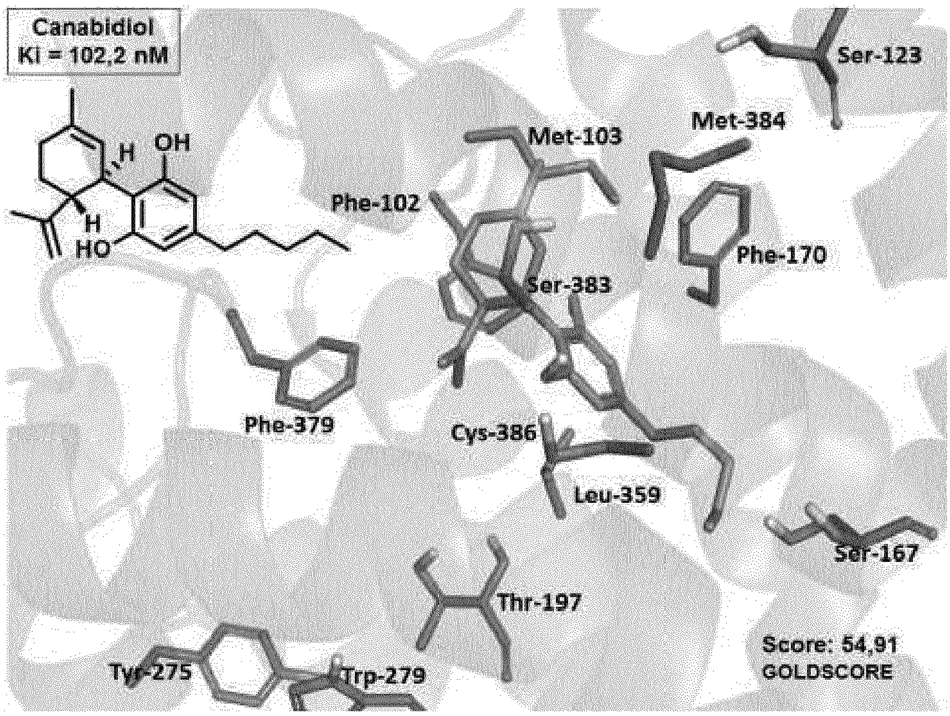
Canabidiol
Ki = 102,2 nM
Met-103
Met-384
Ser-123
Phe-102
Phe-170
Ser-383
Phe-379
Cys-386
Leu-359
Ser-167
Thr-197
Score: 54,91
GOLDSCORE
Tyr-275
Trp-279

Fig. 18

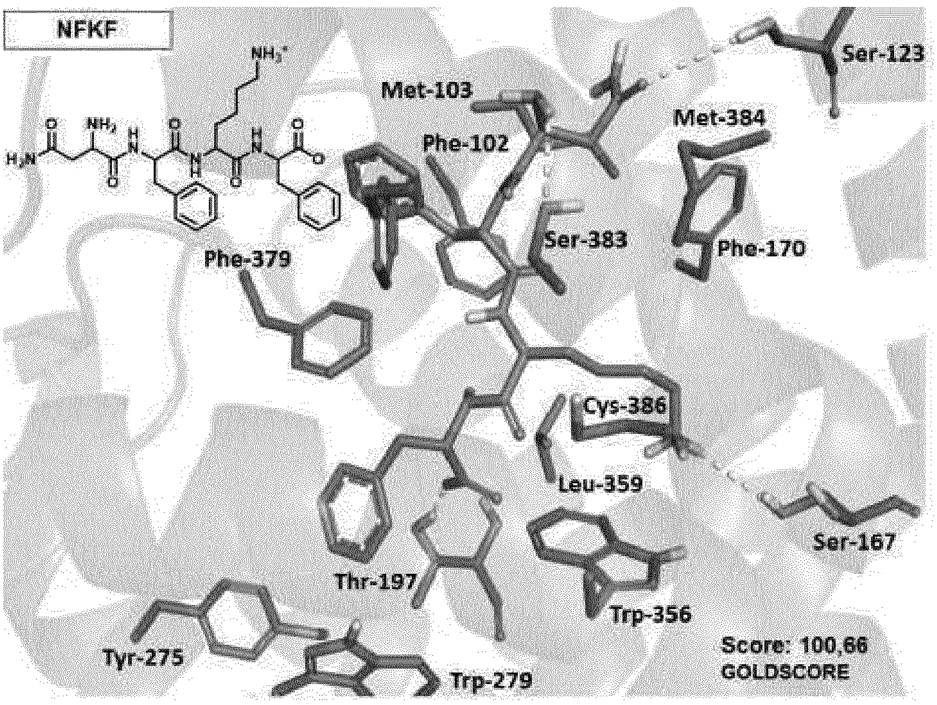
NFKF
Ser-123
Met-103
Met-384
Phe-102
Ser-383
Phe-170
Phe-379
Cys-386
Leu-359
Ser-167
Thr-197
Trp-356
Tyr-275
Score: 100,66
GOLDSCORE
Trp-279

Fig. 19

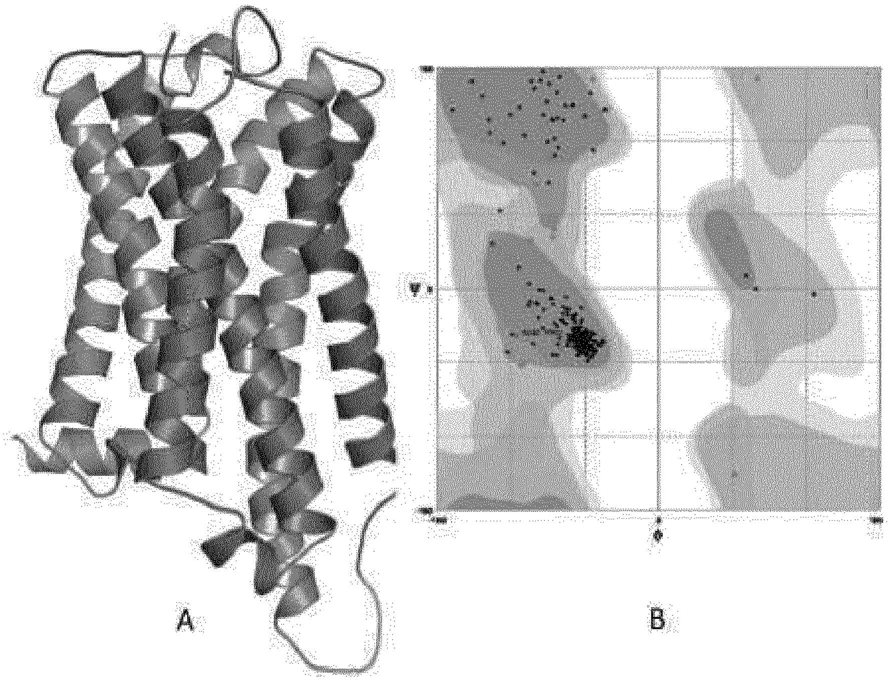
A
B

Fig. 20

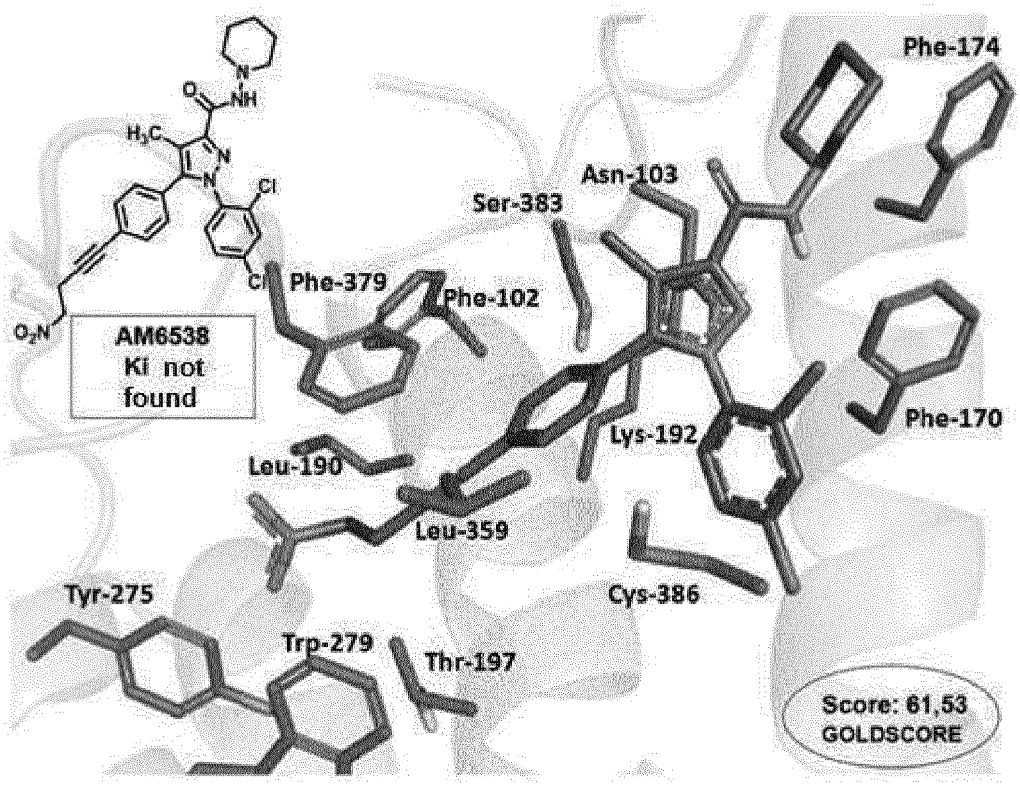
Phe-174
Asn-103
Ser-383
Phe-379
Phe-102
AM6538
Ki not
found
Phe-170
Lys-192
Leu-190
Leu-359
Tyr-275
Cys-386
Trp-279
Thr-197
Score: 61,53
GOLDSCORE

Fig. 21

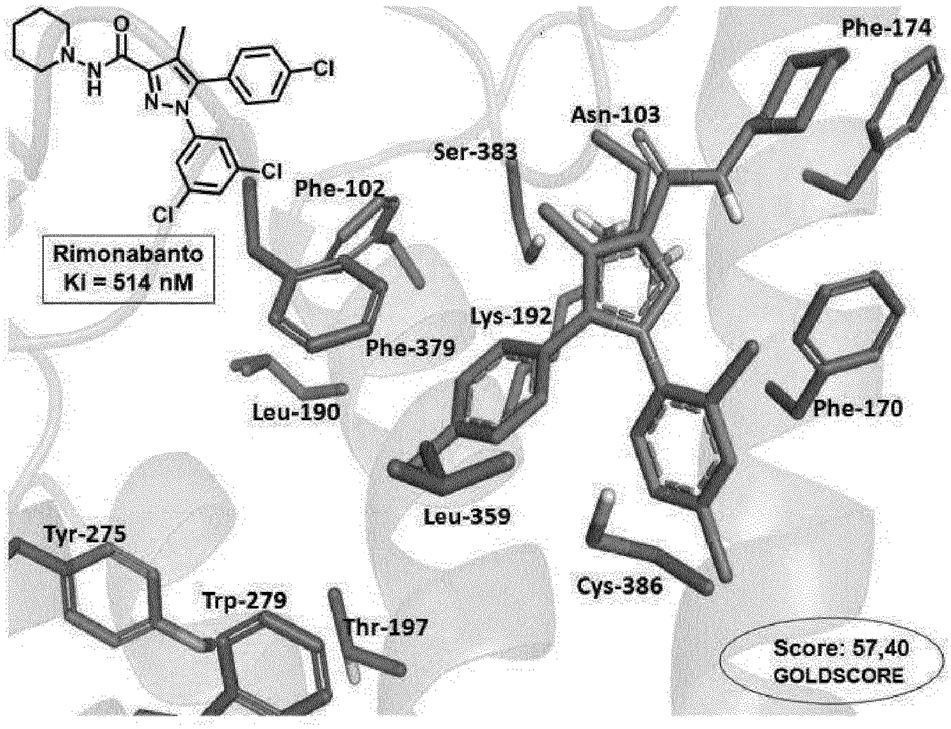
Rimonabanto
Ki = 514 nM
Phe-174
Asn-103
Ser-383
Phe-102
Lys-192
Phe-379
Leu-190
Phe-170
Leu-359
Tyr-275
Cys-386
Trp-279
Thr-197
Score: 57,40
GOLDSCORE

Fig. 22

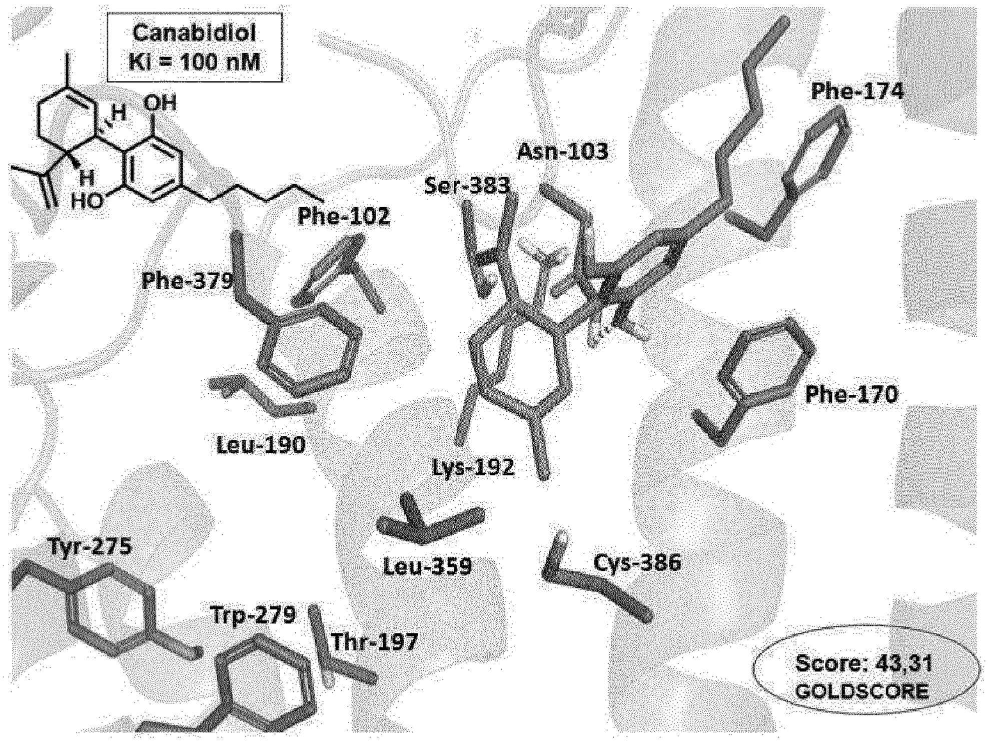
Canabidiol
Ki = 100 nM
Phe-174
Asn-103
Ser-383
Phe-102
Phe-379
Phe-170
Leu-190
Lys-192
Tyr-275
Leu-359
Cys-386
Trp-279
Thr-197
Score: 43,31
GOLDSCORE

Fig. 23

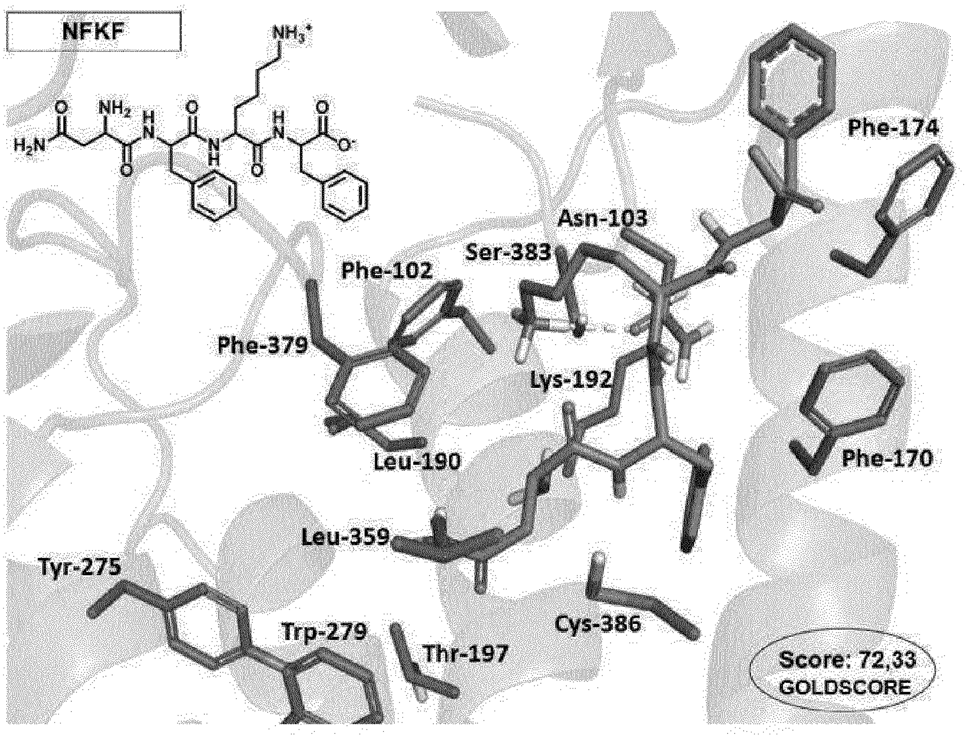
NFKF
Phe-174
Asn-103
Ser-383
Phe-102
Phe-379
Lys-192
Phe-170
Leu-190
Leu-359
Tyr-275
Trp-279
Thr-197
Cys-386
Score: 72,33
GOLDSCORE

Fig. 24

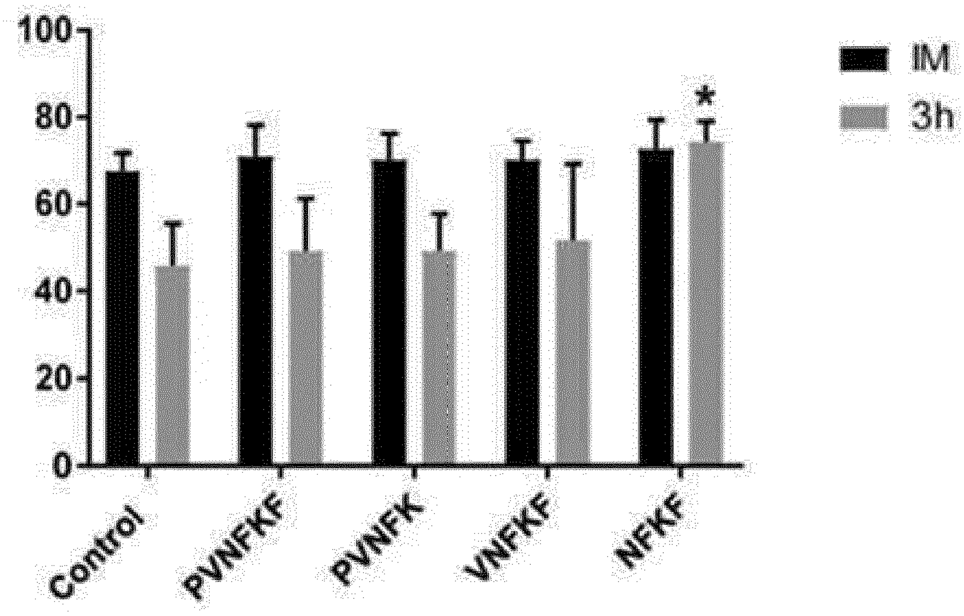
100
80
60
40
20
0
IM
3h
*
Control
PVNFKF
PVNFK
VNFKF
NFKF

Fig. 25

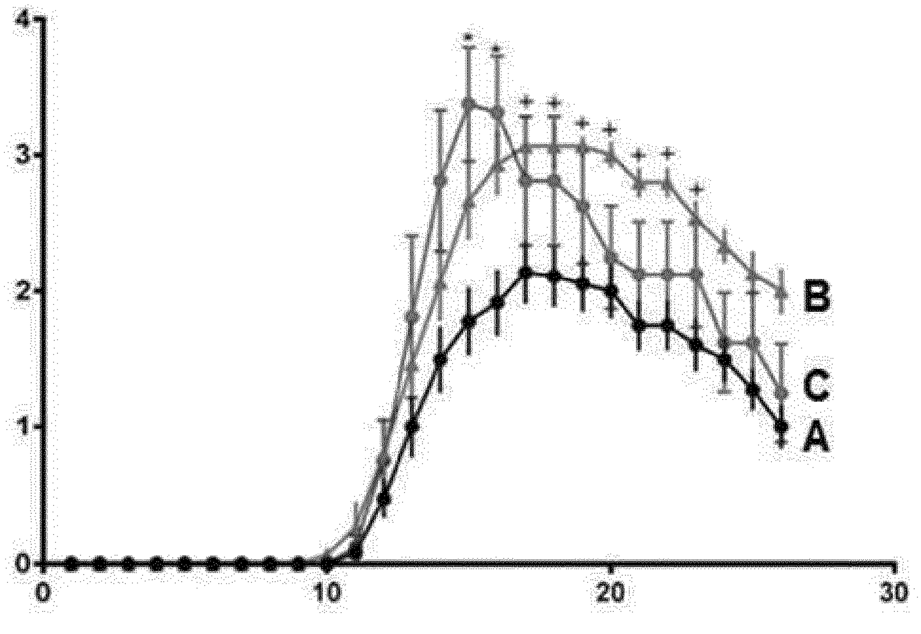

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014008567 A
- WO 2011011847 A
- US 2015297669 A
- US 9452196 B
- EP 2459207 A
- BR 2017050313 W
- WO 2013021196 A


### Non-patent literature cited in the description


- **VANESSA RIOLI** ; **FABIO C. GOZZO** ; **ANDREA S. HEIMANN** ; **ALESSANDRA LINARDI** ; **JOSE E. KRIEGER** ; **CLAUDIO S. SHIDA** ; **PAULO C. ALMEIDA** ; **STEPHEN HYSLOP** ; **MARCOS N. EBERLIN** ; **EMER S. FERRO**. *Novel Peptide Substrates for Endopeptidase 24.15 Neurolysin and Angiotensin Converting Enzyme*, 07 March 2003
- **GOMES I** ; **GRUSHKO JS** ; **GOLEBIEWSKA U** ; **HOOGENDOORN S** ; **GUPTA A** ; **HEIMANN AS** ; **FERRO ES** ; **SCARLATA S** ; **FRICKER LD** ; **DEVI LA**. Novel endogenous peptide agonists of cannabinoid receptors. *FASEB J.*, September 2009, vol. 23 (9), 3020-3029
- **HEIMANN et al.** *Proc Natl Acad Sci USA*, 18 December 2007, vol. 104 (51), 20588-93
- **ASPRONI, B. et al.** Novel pyrrolocycloalkylpyrazole analogues as CB1 ligands. *Chemical Biology and Drug Design*, 2017, 1-13
- **HILDEBRANDT, A. K. et al.** Efficient computation of root mean square deviations under rigid transformations. *Journal of Computational Chemistry*, 2014, vol. 35, 765-771
- **HUA T. et al.** Crystal structure of the human cannabinoid receptor CB1. *Cell*, 2016, vol. 167, 750-762
- **MAIOROV, V. N.** ; **CRIPPEN, G. M.** Significance of root-mean-square deviation in comparing three-dimensional structures of globular proteins. *Journal of Molecular Biology*, 1994, vol. 235, 625-634
- **MORGAN, C. A.** ; **HURLEY, T. D.** Characterization of two distinct structural classes of selective aldehyde dehydrogenase 1A1 inhibitors. *Journal of Medicinal Chemistry*, 2015, vol. 58, 1964-1975
- **PERTWEE, R. G.** The diverse CB1 and CB2 receptor pharmacology of three plant cannabinoids: Δ9-tetrahydrocannabinol, cannabidiol and Δ9 -tetrahydrocannabivarin. *British Journal of Pharmacology*, 2008, vol. 153, 199-215
- **RAMACHANDRAN, G. N.** ; **RAMAKRISHNAN, C.** ; **SASISEKHARAN, V.** Stereochemistry of polypeptide chain configurations. *Journal of Molecular Biology*, 1963, vol. 7, 95-99
- **SANTOS, P. S. et al.** Efeitos do ácido lipóico nas concentrações de glutamato e taurina no hipocampo de ratos após convulsões induzidas por pilocarpina. *Arq. Neuro-Psiquiatr.*, 2011, vol. 69 (2b), 360-364
- **MUNRO et al.** *Nature*, 1993, vol. 365, 61-65
- **RINALDI-CARMONA M. et al.** *J. Pharmacol. Exp. Ther.*, 1996, vol. 278, 871-878
- **GUPTA A** ; **HEIMANN AS** ; **GOMES I** ; **DEVI LA**. Antibodies against G-protein coupled receptors: novel uses in screening and drug development. *Comb. Chem High Throughput Screen.*, 2008, vol. 11 (6), 463-467
- **LANGMEAD CJ1** ; **WATSON J** ; **REAVILL C.** Muscarinic acetylcholine receptors as CNS drug targets. *Pharmacol Ther.*, 20 December 2007, vol. 117 (2), 232-43
- **BOMAR MG** ; **GALANDE AK**. Modulation of the cannabinoid receptors by hemopressin peptides. *Life Sci.*, 2013, vol. 92 (8-9), 520-524
- **DVORÁCSKÓ S** ; **TÖMBÖLY C** ; **BERKECZ R** ; **KERESZTES A**. Investigation of receptor binding and functional characteristics of hemopressin(1-7). *Neuropeptides*, 2016, vol. 58, 15-22
- **GELMAN JS** ; **SIRONI J** ; **CASTRO LM** ; **FERRO, ES** ; **FRICKER LD**. Hemopressins and other hemoglobin-derived peptides in mouse brain: Comparison between brain, blood, and heart peptidome and regulation in Cpefat/fat mice. *J Neurochem.*, May 2010, vol. 113 (4), 871-880
- **BAUER M** ; **CHICCA A** ; **TAMBORRINI M** ; **EISEN D** ; **LERNER R** ; **LUTZ B** ; **POETZ O** ; **PLUSCHKE G** ; **GERTSCH J**. Identification and Quantification of a New Family of Peptide Endocannabinoids (Pepcans) Showing Negative Allosteric Modulation at CB 1 Receptors. *The Journal of Biological Chemistry*, 26 October 2012, vol. 287 (44), 36944-36967

- **REICHWEIN JF** ; **LISKAMP RMJ**. Site-specific N-alkylation of peptides on the solid phase. *Tetrahedron Letters*, 05 March 1998, vol. 39 (10), 1243-1246
- **SZLAVICZ E** ; **PERERA PS** ; **TOMBOLY C** ; **HELYES Z** ; **ZADOR F** ; **BENYHE S** ; **BORSODI A** ; **BOJNIK E**. Further Characterization of Hemopressin Peptide Fragments in the Opioid and Cannabinoid Systems. *Anesth Analg.*, December 2015, vol. 121 (6), 1488-94
- Special Periodic Reports, Amino Acids, Peptides and Proteins. Royal Society of Chemistry, 2013, vol. 42
- Guidance for Industry Estimating the Maximum Safe Starting Dose in Initial Clinical Trials for Therapeutics in Adult Healthy Volunteers. Pharmacology and Toxicology. US Department of Health and Human Services Food and Drug Administration Center for Drug Evaluation and Research (CDER), July 2005